# EUROPEAN PATENT APPLICATION

(11) **EP 4 512 896 A1**
(43) Date of publication of application: **26.02.2025**
(21) Application number: 23791795.0
(22) Date of filing: 14.04.2023
(51) Int. Cl.: C12N 15/09, C07K 16/18, C12M 1/00, C12M 1/34, C12Q 1/68, C12Q 1/6804, C12Q 1/6811, C12Q 1/6844, C12Q 1/686, C12Q 1/6869, C12Q 1/6876, G01N 33/532

(54) **METHOD AND KIT FOR IDENTIFYING MULTIFACTORIAL INTERACTION IN BIOLOGICAL SAMPLE**

(30) Priority: 18.04.2022 JP 2022068519
(71) Applicant: OSAKA UNIVERSITY, Suita-shi Osaka 565-0871 (JP)
(72) Inventor: NIMURA, Keisuke, Suita-shi, Osaka 565-0871 (JP)
(74) Representative: Kraus & Lederer PartGmbB
(86) International application number: PCT/JP2023/015115
(87) International publication number: WO 2023/204147

(57) **Abstract**

The present invention provides a method for identifying multifactorial interactions in a biological sample, comprising (a) selecting a plurality of analytical target factors, (b) labeling antibody molecules against each of the selected factors with DNA barcodes, (c) contacting a biological sample containing the analytical target factors with the antibody molecules labeled with the DNA barcodes, (d) embedding the biological sample in a hydrogel after step (c), (e) performing, within the hydrogel, a nucleic acid amplification reaction using a plurality of primers designed such that, when two antibodies come in close proximity, the primers produce an amplification product containing both DNA barcode sequences from the antibodies, and (f) analyzing a nucleotide sequence of the produced amplification product to detect a presence of a factor that simultaneously interacts with two or more factors.

## Description

### TECHNICAL FIELD

The present invention relates to a method and a kit for identifying multifactorial interactions in a biological sample.

### BACKGROUND ART

Various factors interact with complexity in living bodies, and cells respond to the stimuli and change their phenotypes. Such interactions among multiple factors have been conventionally investigated mainly using cultured cells. Such investigations have been carried out by techniques including biochemical analysis, such as immunoprecipitation, or microscopic analysis using FRET probes. However, these techniques require a large quantity of cells or the expression of a tagged protein. There has been no method that can conveniently analyze multifactorial interactions formed by cells or tissues present in living bodies.

Specific molecular-level information on what types of intermolecular interactions occur in living bodies or how cells communicate with each other have not been fully elucidated. For example, in tumor tissues, multiple factors are involved in various signaling pathways that function within cells and are related to tumor growth and refractory to therapy, such as MYC, RAS, PI3K, WNT, HIPPO, or NOTCH. The factors involved in these pathways exhibit their function through complex interactions with multiple other molecules. However, there is no established method for detecting such multifactorial interactions in a very small amount of cells or tissues. In tumor microenvironments, various types of cells, including T cells, B cells, dendritic cells, macrophages, neutrophils, monocytes, fibroblasts and vascular endothelial cells, interact with each other through receptor-ligand bindings to regulate their active state. There are also needs for development of a method for detecting such interactions in these cells. Analysis of such intracellular or extracellular multifactorial interactions will contribute to establishment of a novel diagnostic method that has not been reported yet.

Assessment of specimens from patients, such as tumor tissues, is currently done by histological staining or genomic analysis using next-generation sequencing. For example, diagnostic methods for investigating genomic instability and mutations have been used for clustering of clinical cases in terms of efficacy of an immune checkpoint inhibitor. However, the conventional methods are unable to quantitatively analyze the degree of interactions between PD-1 and PD-L1 in tumor tissues from a small amount of tissues harvested from patients. Further, factors, such as MYC, that control refractory to therapy or metastasis and growth of tumors interact with other factors not by one-to-one relationship but rather by complicated relationship. There is also no method for evaluating such multifactorial interactions.

A technique for converting the amount of antibody bindings into sequencing information by attaching DNA to antibodies has recently been developed. For example, CITE sequencing is a method for single cell RNA sequencing that involves simultaneously obtaining information on the amount of antibody binding (Non Patent Literature 1). Several methods for measuring interactions between proteins in vitro by sequencing have also been reported, such as a method for quantifying proteins by joining DNA barcodes attached to the proteins (Patent literature 1), or a method for quantifying the amount of binding of a recombinant protein using DNA barcodes attached to the protein (Non Patent Literature 2 to 4). However, these methods are unable to analyze interactions that occurs among multiple factors, and are considered not to be usable for analysis in cells or tissues.

DNA microscopy has been developed as a way to analyze interactions between RNA molecules in cells, and has been previously reported (Non Patent Literature 5). In this technique, RNA molecules are amplified by PCR using primers attached to a polymer, then the PCR product is analyzed by next-generation sequencing, and an image like microphotographs is obtained.

### CITATION LIST

### PATENT LITERATURE

Patent literature 1: JP 2020-502255 A

### NON PATENT LITERATURE

Non patent literature 1: Stoeckius M, Hafemeister C, Stephenson W, Houck-Loomis B, Chattopadhyay PK, Swerdlow H, Satija R, Smibert P. Nat Methods. (2017) 14(9), 865-868.
Non patent literature 2: Yazaki J, Kawashima Y, Ogawa T, Kobayashi A, Okoshi M, Watanabe T, Yoshida S, Kii I, Egami S, Amagai M, Hosoya T, Shiroguchi K, Ohara O. Nucleic Acids Res. (2020) 48(2), e8.
Non patent literature 3: Fredriksson S, Gullberg M, Jarvius J, Olsson C, Pietras K, Gustafsdottir SM, Ostman A, Landegren U. Nat Biotechnol. (2002) 20(5), 473-477.
Non patent literature 4: Nong R, Wu D, Yan J, Hammond M, Gu GJ, Kamali-Moghaddam M, Landegren U, Darmanis S. Nat Protoc. (2013) 8(6), 1234-1248.
Non patent literature 5: Weinstein JA, Regev A, Zhang F. Cell. (2019) 178, 229-241.

### SUMMARY OF INVENTION

### TECHNICAL PROBLEM

An object of the present invention is to provide a method for identifying multifactorial interactions in a biological sample by analyzing the nucleotide sequences of amplification products produced by a single nucleic acid amplification reaction.

### SOLUTION TO PROBLEM

The present invention was made to solve the above problems and includes the following.
[1] A method for identifying multifactorial interactions in a biological sample, comprising the following steps (a) to (f):
   (a) selecting a plurality of analytical target factors,
   (b) labeling antibody molecules against each of the selected factors with DNA barcodes,
   (c) contacting a biological sample containing the analytical target factors with the antibody molecules labeled with the DNA barcodes,
   (d) embedding the biological sample in a hydrogel after step (c),
   (e) performing, within the hydrogel, a nucleic acid amplification reaction using a plurality of primers designed such that, when two antibodies come in close proximity, the primers produce an amplification product containing both DNA barcode sequences from the antibodies, and
   (f) analyzing a nucleotide sequence of the produced amplification product to detect a presence of a factor that simultaneously interacts with two or more factors.
[2] The method according to the above [1], wherein the DNA barcodes each comprise a sequence for distinguishing different antibody types or distinguishing different antibody types and different DNA barcode types, and a sequence for distinguishing individual antibody molecules.
[3] The method according to the above [1], wherein the plurality of primers each comprise a primer set for joining the DNA barcode sequences of two antibodies that come in close proximity, and a primer set for amplifying the joined DNA barcode sequences.
[4] The method according to the above [3], wherein the primers of the primer sets for the joining each comprise a 5' end sequence that hybridizes to its paired primer, a 3' end sequence that hybridizes to a 3' region of one of the DNA barcode sequences, and a sequence for distinguishing individual primer molecules.
[5] The method according to the above [3], wherein the primers of the primer set for the amplification each comprise a sequence that hybridizes to a 5' region of one of the DNA barcode sequences.
[6] The method according to the above [3], wherein the primer set for the joining is immobilized on a polymer that forms the hydrogel.
[7] The method according to any one of the above [1] to [6], wherein the nucleic acid amplification reaction is performed on a glass slide.
[8] The method according to any one of the above [1] to [6], wherein the nucleic acid amplification reaction is PCR.
[9] The method according to any one of the above [1] to [6], wherein the analysis of the nucleotide sequence of the amplification product is performed using a next-generation sequencer.
[10] The method according to the above [9], wherein the primers are designed to produce an amplification product that is shorter than a maximum read length of the next-generation sequencer to be used.
[11] The method according to any one of the above [1] to [6], wherein the biological sample is a human biological sample.
[12] A kit for identifying multifactorial interactions in a biological sample, comprising DNA barcode-labeled antibodies against analytical target factors; primers designed to produce an amplification product formed from joined DNA barcode sequences from two antibodies by a nucleic acid amplification reaction; and a hydrogel.

### ADVANTAGEOUS EFFECTS OF INVENTION

The present invention provides a method for identifying multifactorial interactions in a biological sample by analyzing the nucleotide sequences of amplification products produced by a single nucleic acid amplification reaction. The method of the present invention is able to quantitatively analyze multifactorial interactions in a very small amount of tissue.

### BRIEF DESCRIPTION OF DRAWINGS

Fig. 1 shows the summary of Example 1.
Fig. 2-1 shows the structure of a sequence read of Example 1.
Fig. 2-2 shows the structure of the sequence read of Example 1, showing the left half of the structure of Fig. 2-1 in the upper row and the right half of Fig. 2-1 in the bottom row.
Fig. 3 shows gel electrophoresis of joining PCR amplification products of DNA barcodes after purification. DW indicates the result of water as negative control. Posi indicates positive control. Cell only indicates the result of joining PCR only using cells, oligo only indicates the result of joining PCR using DNA barcodes in the presence of cells, and Ab-oligo indicates the result of joining PCR using DNA barcode-labeled antibodies in the presence of cells.
Fig. 4 shows the results of analysis of the joining PCR products of purified DNA barcodes by next-generation sequencing.
Fig. 5 shows a network diagram showing interactions among LPS, Caspase-11, CD14 and TLR4. The dot size is proportional to the number of sequencing reads.
Fig. 6 shows glass slides used in Example 2. After cells were seeded in the wells, frame-seal slide chambers were placed on the wells, and a hydrogel containing primers was added to the chambers.
Fig. 7 shows microphotographs showing cell morphologies before and after PCR reaction.
Fig. 8 shows gel electrophoresis of joining PCR amplification products of DNA barcodes after purification. Panel A shows the results of joining PCR in the absence or presence of cells. Panel B shows the results of joining PCR using antibodies that bind to analytical target factors (Pol2S2P and H3K27ac) or IgG control in the presence of 5000 cells. The arrows indicate bands with the size of interest.
Fig. 9 shows the results of analysis of the joining PCR products of purified DNA barcodes by next-generation sequencing. The vertical axis indicates the number of sequencing reads.
Fig. 10 shows a network diagram showing interactions among Pol2S2P, Lamin B1, cMYC, H3K27ac and p300 after LPS administration. The dot size is proportional to the number of sequencing reads.

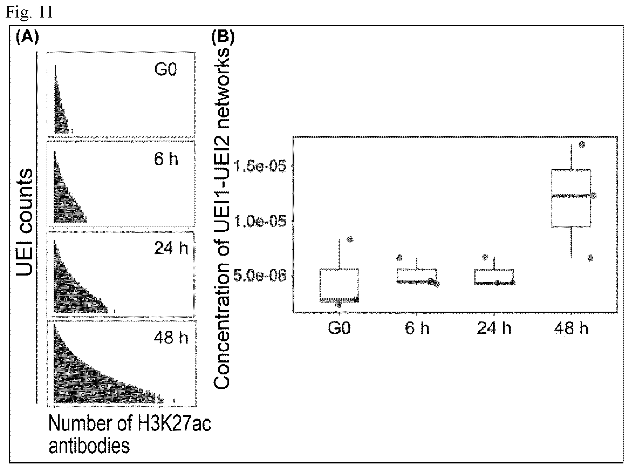

Fig. 11 shows the results of Example 3. Panel A shows the analytical results of the number of bindings of the anti-acetylated histone (H3K27ac) antibody molecules over time per sequence (UEI) for distinguishing individual primer molecules. The vertical axis indicates the UEI counts, the horizontal axis indicates the number of the anti-H3K27ac antibody molecules bound to a single UEI. Panel B shows the results of analysis of the concentration of networks over time, which was assessed as the concentration of networks formed by UEIs, and determined by dividing the observed number of UEI1-UEI2 bindings by the theoretically possible number of UEI1-UEI2 bindings (the observed number of bindings/the theoretically possible number of bindings).
Fig. 12 shows the correlation of the multifactorial interactions detected in Example 3. The dot size is proportional to the number of antibody bindings per UEI. Correlation intensities were indicated by color scales, in which blue represents a correlation coefficient of zero, red represents a correlation coefficient of 1, and white represents a correlation coefficient of a mid-value (0.5).
Fig. 13 shows gel electrophoresis of joining PCR amplification products of DNA barcodes after purification in Example 4. A band of the size of interest was detected after joining PCR using an antibody that binds to an analytical target factor (HLA) in the presence of tissue.
Fig. 14 shows the results of analysis of the joining PCR products of the purified DNA barcodes by next-generation sequencing in Example 4. The graphs show interactions detected between a membrane protein (HLA-DR) and 20 types of analytical target factors in a human renal cancer tissue (A) and a human normal renal tissue (B). The vertical axis indicates the number of sequencing reads.

### DESCRIPTION OF EMBODIMENTS

### Identification method of multifactorial interactions

The present invention provides a method for identifying multifactorial interactions in a biological sample (also referred to as the "method of the present invention" below). The method of the present invention is also called a method for detecting multifactorial interactions in a biological sample, or a method for analyzing multifactorial interactions in a biological sample. The method of the present invention includes the following steps (a) to (f):
(a) selecting a plurality of analytical target factors,
(b) labeling antibody molecules against each of the selected factors with DNA barcodes,
(c) contacting a biological sample containing the analytical target factors with the antibody molecules labeled with the DNA barcodes,
(d) embedding the biological sample in a hydrogel after step (c),
(e) performing, within the hydrogel, a nucleic acid amplification reaction using a plurality of primers designed such that, when two antibodies come in close proximity, the primers produce an amplification product containing both DNA barcode sequences from the antibodies, and
(f) analyzing a nucleotide sequence of the produced amplification product to detect a presence of a factor that simultaneously interacts with two or more factors.

The term "identifying multifactorial interactions" when used in relation to the method of the present invention does not mean identifying an one-to-one interaction between analytical target factors, but rather means identifying simultaneous interactions of at least one analytical target factor with two or more factors. Preferably, the term means identifying multifactorial interactions among multiple selected analytical target factors through comprehensive analysis.

The multifactorial interactions formed by the analytical target factors to be identified in the method of the present invention may be specific interactions or non-specific interactions. Such interactions may be formed by, for example, any one of electrostatic interactions, van der Waals force, hydrogen bonding, coordination bonding, charge transfer interactions, hydrophobic interactions, dipole-dipole interactions, or a combination thereof.

The biological sample used in the method of the present invention is not limited to a particular one, and may be, for example, a portion of a living body harvested from an animal or plant, or a sample prepared from cultured cells. Specific examples of the biological sample include animal or plant tissues, disrupted tissue solutions, extracts, body fluid, excrement, cells, bacteria, viruses, etc. The biological sample used in the method of the present invention is preferably a sample containing cells. The biological sample may also be an unfixed or unfrozen sample, a frozen sample, or a fixed sample using a fixative such as formaldehyde. For example, the biological sample may be cells cultured in any culture vessel and/or on any solid support, or may be a tissue section. The tissue section may be a frozen tissue section or an embedded tissue section in, for example, paraffin etc. The biological sample may also be a clinical specimen, and is preferably a human clinical specimen.

Detection of the interactions in the method of the present invention is achieved by replacing the multifactorial interaction information with DNA molecule binding, and then amplifying the DNA binding, and thus the method can be performed using a small amount of a biological sample. The number of cells that is required to perform the method of the present invention is one or more cells, and may be 10 cells, 100 cells, 500 cells, 1000 cells, 2000 cells, 3000 cells, 4000 cells, or 5000 cells or more.

In step (a), a plurality of analytical target factors are selected. The analytical target factors in the method of the present invention may be any factors that are present in a biological sample, and may be endogenous factors or exogenous factors. The analytical target factors may be naturally occurring low molecular weight organic molecules, naturally occurring organic polymers, low molecular weight synthetic organic molecules, synthetic organic polymers, low molecular weight inorganic molecules, inorganic polymers, organometallic molecules, organic-inorganic hybrid molecules, etc. The analytical target factors are 3 or more types in the method of the present invention, and may be 4 types, 5 types, 10 types, 20 types, 30 types, 40 types, or 50 types or more. The upper limit of the number of types is not particularly limited, and may be 100 types or less, or may be 90 types or less, 80 types or less, 70 types or less, or 60 types or less.

Specific examples of the analytical target factors in the method of the present invention include, but are not limited to, peptides, amino acids, etc.; lipids, such as fatty acids, simple lipids, phospholipids, etc.; saccharides, such as monosaccharides, polysaccharides, oligosaccharides, etc.; nucleic acids, such as DNA, RNA, polynucleotides, oligonucleotides, etc.; glycolipids, lipoproteins, glycoproteins, etc.; proteins, such as extracellular proteins, cell surface proteins, enzymes, receptors, major histocompatibility complexes, tumor antigens, cell adhesion factors (integrins etc.), intracellular proteins, etc.; hormones, cytokines, lymphokines, neurotransmitters, endocrine disruptors, organic compounds, inorganic compounds, toxins, bacteria, viruses; etc.

In step (b), antibody molecules against each of the analytical target factors selected in step (a) are labeled with DNA barcodes. The term "antibody" as used herein includes an antibody fragment that maintains an ability to specifically bind to an antigen. The antibodies used in the method of the present invention may be any antibodies that are capable of binding to the analytical target factors selected in step (a). The antibodies used in the method of the present invention may be naturally occurring antibodies, full-length immunoglobulin molecules of any isotypes formed by recombinant process of immunoglobulin gene fragments, or antibody fragments containing an antigen binding portion of an immunoglobulin molecule. The antibody fragments containing an antigen binding portion of an immunoglobulin molecule may be, for example, F(ab')₂, Fab', Fab, Fv, scFv, etc. The antibodies used in the method of the present invention may be monoclonal antibodies or polyclonal antibodies, and are preferably monoclonal antibodies. The antibodies used in the method of the present invention may be chimeric antibodies or humanized antibodies.

The antibodies used in the method of the present invention can be produced by a method well-known to a person skilled in the art. For example, blood including serum, ascites fluid, egg yolk, or the like collected from an animal immunized with an antigen may be used, or such blood including serum, ascites fluid, egg yolk, or the like may be purified and then used, or the antibodies may be produced using phage display technique etc. The antibodies used in the method of the present invention may be the culture supernatant of hybridomas available from a known cell bank or hybridoma bank, or such culture supernatant may be purified and then used, or the antibodies may be obtained from a antibody bank, or may be commercially available antibodies.

The term "DNA barcode" as used herein refers to a nucleic acid sequence called a tag (JP H01-507357 A, JP 2002-518060 A), a ZIP code (JP 2001-519648 A), a normalized orthogonal sequence (JP 2002-181813 A), a barcode probe (Q. Xu, et al. (2009) PNAS 106(7), 2289-2294), etc.

The DNA barcode used in the method of the present invention preferably contains a sequence for distinguishing different antibody types or distinguishing different antibody types and different DNA barcode types, and a sequence for distinguishing individual antibody molecules. The sequence for distinguishing different antibody types is a sequence that is used to distinguish different antibody types against the plurality of analytical target factors selected in step (a). When a plurality of DNA barcodes are used for a single factor to comprehensively analyze interactions of a plurality of factors, a sequence for simultaneously distinguishing different antibody types and different DNA barcode types is used in place of the sequence for distinguishing different antibody types. The sequence for distinguishing individual antibody molecules is a sequence that is used to distinguish individual single antibody molecules used in the method of the present invention.

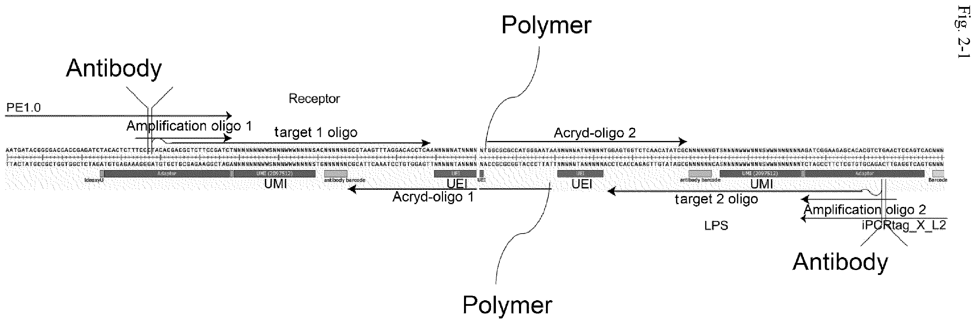

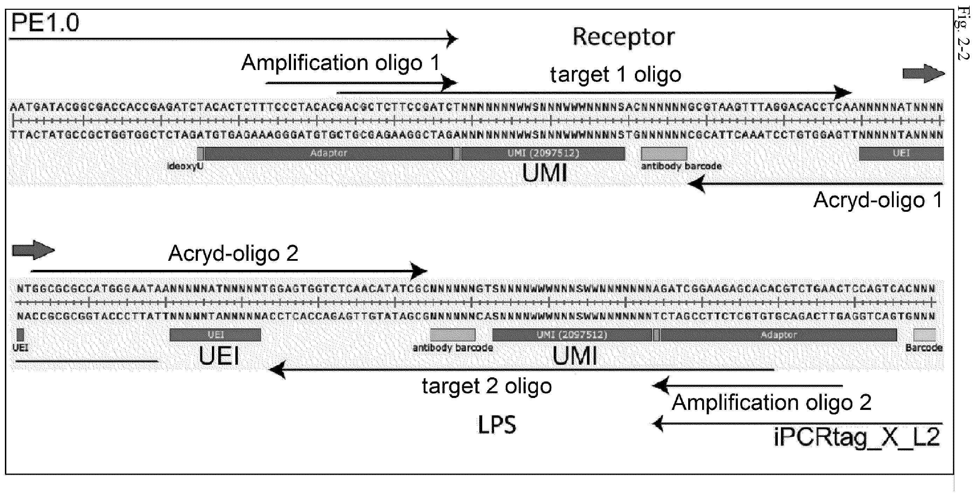

Specific examples of the DNA barcodes used in the method of the present invention include target 1 oligo and target 2 oligo as shown in Figs. 2-1 and 2-2. In target 1 oligo and target 2 oligo, the sequences for distinguishing different antibody types or for distinguishing different antibody types and different DNA barcode types are the sequences in the regions indicated by "antibody barcode," and the sequences for distinguishing individual antibody molecules are the sequences in the regions indicated by "UMI." The sequences for distinguishing individual antibody molecules are preferably designed not to include a contiguous sequence of a single type of nucleotides. UMI (Unique Molecular Identifier) can be designed based on, for example, a previous report (J. A. Weinstein et al., Cell, 2019, 178:229-241.e1-e10).

The length (the number of nucleotide residues) of the sequences for distinguishing different antibody types or for distinguishing different antibody types and different DNA barcode types, and the length (the number of nucleotide residues) of the sequences for distinguishing individual antibody molecules are not limited to a particular one, and may be designed as appropriate. When a next-generation sequencer is used in step (f), the DNA barcodes are preferably designed such that the nucleotide length of an amplification product containing two joined DNA barcodes is shorter than the maximal read length of the sequencer used. Also when a next-generation sequencer is used in step (f), the DNA barcodes may be designed to contain adapter sequences appropriate for the sequencer used. Further, the 3' regions of the DNA barcodes preferably contain a sequence to which the primers used in step (e) hybridize.

Labeling of the antibody molecules with the DNA barcodes may be done by any method that is well-known to a person skilled in the art. For example, a functional group, such as an amino acid, an aldehyde group, an SH group or biotin, is previously introduced into one end of the DNA barcodes, and simultaneously, a functional group that can react to the oligonucleotide, such as an aldehyde group, an amino group, an SH group, or streptavidin is introduced into the antibodies, and then the functional groups are allowed to covalently interact with each other to form crosslinks, thereby labeling the antibodies with the DNA barcodes. Alternatively, the antibodies may be labeled with the DNA barcodes using a commercially available crosslinking agent (crosslinker) or a commercially available labeling kit. Preferred is labeling by biotin-streptavidin binding. Preferably, labeling is performed such that the antibodies are bound to the 5' end of the DNA barcodes.

In step (c), a biological sample containing the analytical target factors is allowed to contact with the antibody molecules labeled with the DNA barcodes. The contact between the biological sample and the DNA barcode-labeled antibody molecules may be done by any method, including, for example, but not limited to, immersing the biological sample in an antibody solution; layering an antibody solution over the biological sample; adding the antibodies to a culture system of the biological sample; or other methods. The biological sample may be loaded onto a solid support and allowed to contact with the antibodies. The solid support may be any solid support that can be loaded with a biological molecule, and examples of such solid support include a filter, a membrane, a microwell plate, a bead, a slide, etc. The solid support may be made of a material such as glass, a plastic, a fluororesin, ceramics, a metal, silica, methylstyrene, latex, Sepharose, agarose, cellulose, a polymer material, etc. Preferred are a glass bead or a glass slide among them, preferably a glass slide. By loading the biological sample onto a solid support, the loss of cells is prevented. Thus, the method of the present invention is able to identify multifactorial interactions even in a small amount of the biological sample.

When a glass slide is used as a solid support, the glass slide is preferably capable of allowing culture of the biological sample and/or retaining the biological sample thereon and is then capable of using for a nucleic acid amplification reaction. When the PCR method is used in step (e), the glass slide preferably has a size appropriate for a PCR device used. The glass slide may be provided with coating of, for example, APS, MAS or poly-L-lysine on the surface. The glass slide may be provided with special printing using silver stain, an ultraviolet curing ink, a fluororesin ink, etc., or provided with hydrophobic printing etc. The glass slide, when used in the method of the present invention, may be used together with a chamber, such as a culture medium chamber or a frame-seal chamber.

When the biological sample contains cells, the cells may be permeated before contact with the antibodies. In particular, when the analytical target factors are located within cells, the cells are preferably permeated before contact with the antibodies. Permeation of the sample may be done by any method that is well-known to a person skilled in the art. For example, the biological sample may be fixed in a fixative, such as formaldehyde, and then the cells may be permeated with a buffer solution containing a surfactant, such as TritonX-100. Alternatively, a reagent that is capable of simultaneously performing fixation and permeation may be added to an unfixed biological sample. The reagent that is capable of simultaneously performing fixation and permeation may be a cold alcohol etc. Blocking for preventing a non-specific antibody reaction may be performed on the biological sample before contact with the antibodies. Blocking of the sample may be done by any method that is well-known to a person skilled in the art.

The biological sample that has been contacted with the antibodies may be post-fixed. Post-fixation may be performed in a known fixative such as formalin (aqueous formaldehyde solution). If necessary, the biological sample that has been contacted with the antibodies may be contacted with the antibodies again. For example, the biological sample contacted with the antibodies without permeation may be permeated, and then contacted with the antibodies again to allow analysis of both of factors localized on the surface of the cell membrane and factors localized within the cells as analytical targets.

**In** step (c), the DNA barcode-labeled antibody molecules may first be subjected to fluorescent labeling, enzyme labeling, etc., and then contacted with the biological sample. Alternatively, the DNA barcode-labeled antibody molecules may first be contacted with the biological sample, and then contacted with secondary antibodies, such as fluorescence-labeled antibodies or enzyme-labeled antibodies. **In** particular, for example, when secondary antibodies are used, the DNA barcode-labeled antibody molecules may first be contacted with the biological sample loaded on a glass slide, and then immunostained with fluorescence-labeled antibodies, and subjected to the following step (d). **In** step (d), the biological sample is embedded in a hydrogel, and observed under a fluorescent microscope to capture an image, and then a nucleic acid amplification reaction is performed in step (e) to simultaneously obtain image information, such as the morphology of the cells or the localization of the target factors, and the nucleotide sequence information of the amplification products.

In step (d), the biological sample that has been contacted with the DNA barcode-labeled antibodies in step (c) is embedded in a hydrogel. The hydrogel used in the method of the present invention is not limited to a particular one. The hydrogel may be, for example, a swelled body formed by water-induced swelling of a three-dimensional network of crosslinked polymer chains, or may be a hydrogel formed by water-induced swelling of a structure formed by crosslinking between different molecules of polymers, such as water-soluble polysaccharides or proteins. The polymers are not limited to a particular one, and may be a naturally occurring polymer, a synthetic polymer, or a combination thereof. Examples of the hydrogel include collagen, hyaluronic acid, chitosan, fibrin, gelatin alginate, agarose, chondroitin sulfate, polyacrylamide, polyethylene glycol (PEG), polyvinyl alcohol (PVA), polyacrylamide/polyacrylic acid (PAA), hydroxyethyl methacrylate (HEMA), poly(N-isopropylacrylamide) (NIPAM), a polyanhydride, polypropylene fumarate (PPF), etc. Preferred is a hydrogel made of PEG. When a hydrogel made of PEG is used, the PEG may be multi-arm PEG, PEG modified with a functional group, such as a thiol group, or a combination thereof. Preferably, the hydrogel is a combination of multi-arm PEG and thiol-group modified PEG. More preferably, the hydrogel is a combination of 4-Arm PEG and 2SH PEG. Both of 4-Arm PEG and 2SH PEG are commercially available from suppliers. For example, when a combination of 4-Arm PEG and 2SH PEG is used in the method of the present invention, a 4-Arm PEG solution and a 2SH PEG solution can separately be prepared in ultrapure water each at 500 µg/µL, and the solutions can then be combined to initiate gelation to prepare a hydrogel.

For example, in step (c), a small number of cells may be loaded on a glass slide, and a biological sample that has been contacted with the DNA barcode-labeled antibodies may be embedded in a hydrogel. Then, after the location of the cells are identified, a single cell may be recovered together with the hydrogel, and subjected to the following step (e). In this manner, multifactorial interactions in a single cell can be identified.

In step (e), a nucleic acid amplification reaction is performed within the hydrogel by using a plurality of primers designed such that, when two antibodies come in close proximity, the primers produce an amplification product containing both DNA barcode sequences from the antibodies. The nucleic acid amplification reaction used in step (e) may be any nucleic acid amplification reaction that can produce such an amplification product, and may be selected from known nucleic acid amplification reactions. Specific examples of the nucleic acid amplification reaction include PCR (Polymerase Chain Reaction) method, LAMP (Loop-Mediated Isothermal Amplification) method, WGA (Whole Genome Amplification) method, MDA (Multiple Displacement Amplification) method, HDA (Helicase-dependent amplification) method, TRC (Transcription Reverse Transcription Concerted Reaction) method, RPA (Recombinase Polymerase Amplification) method, SIBA (Strand-Invasion Based Amplification) method, ICAN (Isothermal and Chimeric primer-initiated Amplification of Nucleic acids) method, LCR (Ligase Chain Reaction) method, SDA (Strand Displacement Amplification) method, NEAR (Nicking Endonuclease Amplification Reaction) method, SMAP (Smart amplification process) method, 3SR (Self-stranded sequence replication) method, NASBA (Nucleic Acid Sequence-Based Amplification) method, TMA (Transcription-Mediated-Amplification) method, etc. The nucleic acid amplification reaction is preferably the PCR method, the LAMP method, the HDA method or the SMAP method, more preferably the PCR method or the LAMP method, and further preferably the PCR method.

The nucleic acid amplification reaction is performed using primers designed such that, when two antibodies are come in close proximity, the primers produce an amplification products containing both DNA barcode sequences from the antibodies. The primers are designed and used as appropriate for the nucleic acid amplification reaction used. For example, when the PCR method is used as the nucleic acid amplification reaction, primers may be designed to join the DNA barcodes from two antibodies that come in close proximity based on the overlap extension PCR method, and then used for the reaction. For the technique for creating joined DNA fragments (concatemers) using the overlap extension PCR method, reference may be made to, for example, Yolov et al. (A.A. Yolovand Z.A. Shabarova, Nucleic Acids Res., 1990, 18(13):3983-3986) or Turchaninova et al. (M.A. Turchaninova et al., Eur. J. Immunol., 43:2507-2515).

The primers preferably contain a primer set for joining the DNA barcode sequences of two antibodies that come in close proximity, and a primer set for amplifying the joined DNA barcode sequences. The primers of the primer set for joining the DNA barcode sequences of two antibodies that come in close proximity (referred to as the "joining primers" below) preferably contain a 5' end sequence that hybridizes to its paired primer, a 3' end sequence that hybridizes to a 3' region of one of the DNA barcode sequences, and a sequence for distinguishing individual primer molecules. The primers of the primer set for amplifying the joined DNA barcode sequences (referred to as the "amplification primers" below) preferably contain a sequence that hybridizes to a 5' region of one of the DNA barcode sequences. When a next-generation sequencer is used in step (f), the amplification primers may be designed to add adapter sequences to the ends of amplification products.

Specific examples of the primers used in the method of the present invention include Acryd-oligo 1 and Acryd-oligo 2 (joining primers), as well as amplification oligo 1 and amplification oligo 2 (amplification primers), as shown in Figs. 2-1 and 2-2. In Acryd-oligo 1 and Acryd-oligo 2, the sequences distinguishing individual primer molecules are the sequences in the regions indicated by "UEI." UEI (Unique Event Identifier) can be designed based on, for example, a previous report (J. A. Weinstein et al., Cell, 2019, 178:229-241.e1-e10). In step (e), when the produced amplification product contains two joined DNA barcode sequences, the amplification product contains two UEIs each from each of the joining primers.

The joining primers, among the above primers, are preferably immobilized on a polymer that forms the hydrogel. By immobilizing the joining primers to the polymer, multifactorial interactions can be identified while their spatial information is maintained. Immobilization of the joining primers to the polymer can be done by, for example, adding the joining primers having Acridite modification at the 5' end to a polymer solution, and gelling the polymer solution to prepare a hydrogel, but the immobilization method is not limited to this, and may be done by a technique well-known to a person skilled in the art.

When a next-generation sequencer is used in step (f), the joining primers and the amplification primers are preferably designed such that the nucleotide length of an amplification product is shorter than the maximal read length of the sequencer used. Such an amplification product with a nucleotide length shorter than the maximal read length of a sequencer can be subjected to sequencing analysis for identification of interactions in a single read. For example, when an Illumina sequencer is used, the joining primers and the amplification primers are preferably designed such that the nucleotide length of an amplification product containing two DNA barcodes is 150 bp or less.

The nucleic acid amplification reaction can be performed based on a known technique of the nucleic acid amplification reaction used. The reagents, the instruments, and others to be used can be commercially available products. When the nucleic acid amplification reaction is the PCR method, a reaction solution is added to the biological sample that is embedded in a hydrogel to initiate the reaction. The PCR reaction solution contains a nucleic acid synthase (DNA polymerase), nucleotides serving as substrates of the nucleic acid synthase, primers other than the primers immobilized to the hydrogel. The reaction solution also contains a buffering agent, a salt, etc., and if needed, further contain a protective agent for the enzyme, a modifier of Tm, a surfactant, etc. A specific composition of the reaction solution is preferably determined as appropriate by performing preliminary examinations on a particular combination of the biological sample, the DNA barcode-labeled antibodies, the primers, the nucleic acid synthase, etc. used.

The PCR method, when used in the method of the present invention, may be performed by the common three step method (denaturation → annealing → elongation cycles), or the two step method (denaturation → annealing/elongation cycles). The reaction conditions including the denaturation temperature, the annealing temperature, the elongation temperature, and the reaction time of each step are preferably determined as appropriate by performing preliminary examinations on a particular combination of the biological sample, the DNA barcode-labeled antibodies, the primers, the nucleic acid synthase, etc. used.

Extraction of the amplification product from the hydrogel may be done by any method appropriately selected from known extraction methods depending on the hydrogel used and the nucleic acid amplification method used. For example, when a hydrogel formed by a combination of 4-Arm PEG and 2SH PEG is used, a PEG-dissolution solution containing potassium hydroxide is added to the hydrogel after a nucleic acid amplification reaction, and the hydrogel is heated at 72°C to dissolve the hydrogel, and reacted with a protease, such as protease K, thereby extracting the amplification product (see Example 1).

If necessary, the extract may be examined whether the amplification product exists. Detection of the amplification product may be performed by any common method that can detect a nucleic acid, and the method may be a qualitative detection method or a quantitative detection method. Such a detection method may be, for example, agarose gel electrophoresis etc.

In step (f), the nucleotide sequence of the amplification product produced in step (e) is analyzed to detect a presence of a factor that simultaneously interacts with two or more factors. The nucleotide sequence analysis may be done by any known method, including Sanger method (dideoxy method), a method by using a next-generation sequencer, etc. Preferred is a method by using a next-generation sequencer.

For example, when the Sanger method (dideoxy method) is used, the amplification product extracted from the hydrogel can run on agarose gel electrophoresis, then the amplification product can be extracted from the gel, and a sample for sequence analysis can be prepared by a known method and run on a commercially available sequencer to determine the nucleotide sequence.

When a next-generation sequencer is used, the amplification product may be purified. The purification may be done by any method appropriately selected from known purification methods. A commercially available nucleic acid purification kit may be used. When a next-generation sequencer is used, adapter sequences need to be added to the amplification product. The adapter sequences can be added by any method, and may be added by, for example, performing PCR using the amplification product as a template and using a primer set designed to add the adapter sequences. The size of the amplification product with added adapter sequences is preferably within the read length of a next-generation sequencer used.

The obtained nucleotide sequence data may be analyzed using variety of programs developed for sequence data processing. Such programs may be, for example, fastq_quality_trimmer, umi_tools, igraph, etc. When a next-generation sequencer is used, the obtained sequence data are typically subjected to processing steps, including import of the data aggregates from the sequencer, trimming of unnecessary sequence data such as the adapters and the primers, removing of low quality sequence regions, assembly of the reads, etc.

Presence of a factor that simultaneously interacts with two or more factors in the method of the present invention can be detected by discovering an amplification product that contains two DNA barcode sequences, one of which is the same as the DNA barcode sequence for detecting the factor, and the other one is different. Whether the amplification product is resulted from the joining of the DNA barcodes of the antibodies that come in close proximity, i.e., whether the amplification product reflects the interaction of two factors can be determined by the presence of the sequences for distinguishing two individual antibody molecules contained in each DNA barcode (e.g., UMI sequences) and the sequences for distinguishing two individual primer molecules contained in each joining primer (e.g., UEI sequences). Whether the DNA barcode sequences are the same or different can be determined by determining whether the sequences for distinguishing individual antibody molecules (e.g., UMI sequences) are the same or different. For example, when four types of analytical target factors A, B, C and D are selected in step (a), the method of the present invention can be used to identify multifactorial interactions among factors A, B, C and D by detecting whether factor A simultaneously interacts with two or more of B, C and D, whether factor B simultaneously interacts with two or more of A, C and D, whether factor C simultaneously interacts with two or more of A, B and D, or whether factor D simultaneously interacts with two or more of A, B and C.

The method of the present invention can be used for, for example, functional assessment of tissues, which cannot be evaluated by a conventional histological staining or genome diagnosis. In particular, the results obtained from the method of the present invention can be used for prediction of the therapeutic effect of various medicines, clustering of clinical cases, or the like, in clinical diagnosis.

### Kit

The present invention provides a kit for performing the method of the present invention as described above (also referred to as the "kit of the present invention" below). The kit of the present invention contains DNA barcode-labeled antibodies against a plurality of analytical target factors to be subjected to identification of multifactorial interactions; primers designed to produce an amplification product formed from joined DNA barcode sequences from two antibodies by a nucleic acid amplification reaction; and a hydrogel. The DNA barcode-labeled antibodies, the primers and the hydrogel contained in the kit of the present invention may be those described above regarding the embodiments of the method of the present invention. The kit of the present invention may further contain a DNA polymerase required for a nucleic acid amplification reaction, a deoxyribonucleoside triphosphate (dNTP) mixture, and a primer set for adding adapters for next-generation sequencing.

The kit of the present invention may further contain a buffer solution. The buffer solution may be, for example, but is not limited to, a buffer solution for preparation of a reagent, a buffer solution for a nucleic acid amplification reaction, a buffer solution for collecting an amplified DNA, etc. The kit of the present invention may further contain a reagent for purifying an amplified DNA. The kit of the present invention may further contain various types of tubes for preparation of a reagent, a solid support such as a glass bead or a glass slide, instructions for use, etc. The kit of the present invention can be used to perform the method for identifying multifactorial interactions according to the present invention in an easy and rapid manner when specific analytical target factors of interest are known. The kit of the present invention may be implemented as a kit of custom-made. The kit according to the users' purpose of use can be provided by designing the DNA barcode-labeled antibodies and the primer set as appropriate for the purpose of use.

### EXAMPLES

The present invention will be described in more detail below with reference to Examples, but the present invention is not limited thereto.

### Example 1: Identification of interactions between LPS and LPS receptors

### 1. Materials and methods

### 1-1 Production of DNA barcode-conjugated antibodies

### Preparation of biotinylated DNA barcodes

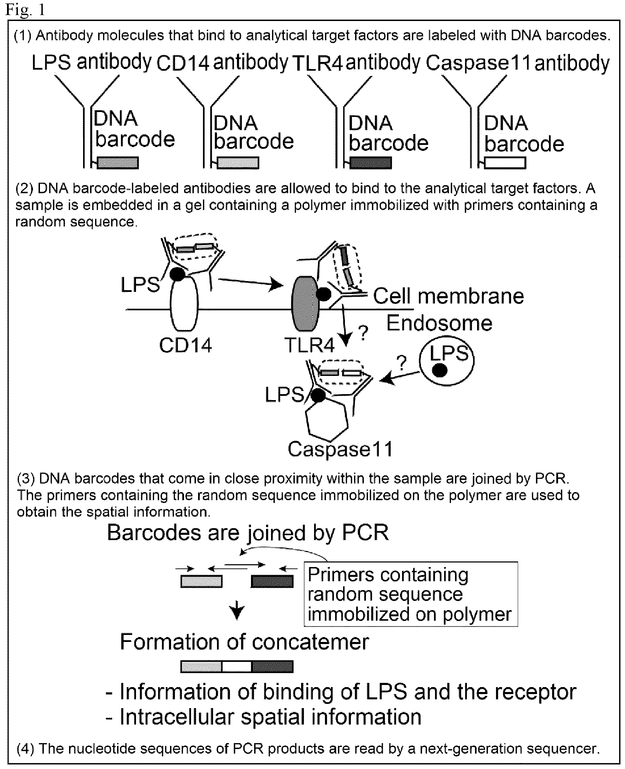

To prepare DNA barcode-conjugated antibodies as schematically shown in Fig. 1, DNA barcodes having antibody barcode sequences (sequences for distinguishing different antibody types), UMI sequences (sequences for distinguishing individual antibody molecules), and adapter sequences for sequencing were designed for each of LPS and factors that are known to interact with LPS, including Caspase-11, CD14 and TLR4. These DNA barcodes, indicated as target 1 oligo and target 2 oligo in Figs. 2-1 and 2-2, were synthesized by a known technique, and the 5' end was biotinylated by a conventional method. Each of the biotinylated DNA barcodes was diluted in Tris-EDTA buffer to 100 µM, heated at 55°C for 10 minutes, and centrifuged at 18000xg at room temperature for 5 minutes. The concentration in the supernatant was checked and adjusted again, if necessary, to 100 µM. The sequences of the DNA barcodes including the UMI sequences and the antibody barcode sequences are shown below. The antibody barcode sequences are underlined.
- UMI: NNNNNNNWWSNNNWWWNNNNS (SEQ ID NO: 1),
- target 1 oligo (5biotin)_BC28 (DNA barcode for anti-Caspase 11 antibody): 
GACGCTCTTCCGATCTNNNNNNNWWSNNNWWWNNNNSACCTTTTGGCGTA
AGTTTAGGACACCTCA (SEQ ID NO: 2),
- target 1 oligo (5biotin)_BC24 (DNA barcode for anti-CD 14 antibody): 
GACGCTCTTCCGATCTNNNNNNNWWSNNNWWWNNNNSACGCTACCGCGTA
AGTTTAGGACACCTCA (SEQ ID NO: 3),
- target 1 oligo (5biotin)_BC26 (DNA barcode for anti-TLR4 antibody): 
GACGCTCTTCCGATCTNNNNNNNWWSNNNWWWNNNNSACGCTCATGCGTA
AGTTTAGGACACCTCA (SEQ ID NO: 4), and
- target 2 oligo (5biotin)_BC1 (DNA barcode for anti-LPS antibody):

TGTGCTCTTCCGATCTNNNNNNNWWSNNNWWWNNNNSACCGTGATGCGAT
ATGTTGAGACCACTCC (SEQ ID NO: 5).

### Preparation of antibody-streptavidin complexes

The solvent of the antibodies below was exchanged with PBS using a centrifugal filter (trade name: Amicon Ultra 0.5, Merck, Cat. No. UFC505096) following the manufacturer's protocol. The antibodies used were as follows: anti-LPS antibody (mouse monoclonal antibody, Abcam, Cat. No. ab35654), anti-CD14 antibody (mouse monoclonal antibody, BioLegend, Cat. No. 123302), anti-TLR4 antibody (mouse monoclonal antibody, BioLegend, Cat. No. 145401), and anti-Caspase 11 antibody (rat monoclonal antibody, SIGMA-Aldrich, Cat. No. C1354). After buffer exchange, the antibody concentrations were adjusted to 15 µg in 30 µL. The antibodies were then labeled with streptavidin using a commercially available labeling kit (trade name: LYNX Rapid Streptavidin Antibody Conjugation Kit, BIO-RAD, Cat. No. LNK163STR). The labeling was performed following the manufacturer's protocol.

### Preparation of DNA barcode-labeled antibodies

The streptavidin labeled-antibodies were allowed to bind to the corresponding biotinylated DNA barcodes to form conjugates. Briefly, 3 µL of quenching solution (included in the labeling kit above) was added to vials containing one of the streptavidin-labeled antibodies, and allowed to stand at room temperature for 30 minutes to quench the reaction. Then, 5 M NaCl and 0.1% Tween20/H₂O, at 4 µL each, were added. The total volume was transferred to a fresh low-binding microtube, and 800 pmol of the corresponding biotinylated DNA barcode was added and reacted at room temperature overnight. The combinations of the antibodies and the DNA barcodes are as follows: anti-Caspase 11 antibody-target 1 oligo (5biotin)_BC28 (SEQ ID NO: 2), anti-CD14 antibody-target 1 oligo (5biotin)_BC24 (SEQ ID NO: 3), anti-TLR4 antibody-target 1 oligo (5biotin)_BC26 (SEQ ID NO: 4), and anti-LPS antibody-target 2 oligo (5biotin)_BC1 (SEQ ID NO: 5). The solvent of the solutions containing one of the DNA barcode-labeled antibodies after the reaction was exchange with PBS using a centrifugal filter (Amicon Ultra 0.5, Merck) by a conventional method, and the total volume was adjusted to 30 µL with PBS.

The DNA barcode-labeled antibodies prepared as above were run by electrophoresis on a 4% TAE gel for 10 minutes. The gel was then treated with ethidium bromide for 10 minutes to visualize the bands to examine the conjugation of the DNA barcodes. As controls, 50 pmol of the DNA barcodes were run, and the relative band intensities were determined for all bands appeared on the lanes of the DNA barcode-labeled antibody samples by image analysis. The amounts of the DNA barcodes in the prepared DNA barcode-labeled antibodies were estimated. Samples for which successful conjugation of the DNA barcodes was confirmed were stored at 4°C after addition of 50 mg/mL BSA and 1% sodium azide at final concentrations of 1 µg/µL and 0.05%, respectively.

### 1-2 Preparation of specimens

Glass beads of 3 mm diameter (Sigma-Aldrich, Cat. No. Z265926-1EA) were inserted into 8-tube PCR strips and were fixed with silicone. The prepared tubes for PCR reaction were rinsed twice with 70% ethanol, and dried under UV irradiation with the lids slid to open. The procedures were performed in a clean bench, and the prepared tubes were stored filled with PBS.

Purified mouse B cells were suspended in serum-free medium, and seeded in the prepared PCR tubes as described above by adjusting the density to 5000 cells/50 µL per tube. The cells were cultured in 5% CO₂ at 37°C for 30 minutes, and the culture supernatant was removed. Then, 1 mg/mL LPS and 2.5 µg/mL IL-4 were diluted in RPMI 1640 medium to final concentrations of 50 µg/mL and 2.5 ng/mL, respectively, and added to the tubes at 50 µL each. The cells were cultured in 5% CO₂ at 37°C for 20 minutes to 48 hours. IL-4 was used as stimulation for B-cell activation. The compositions of the serum-free medium and RPMI 1640 medium are as follows:
Serum-free medium: 29 mL RPMI 1640 (Nacalai Tesque, Cat. No. 30264-85), 300 µL 100x Penicillin/Streptomycin (final 1x), 30 µL 2-ME (final 53 µM), 300 µL 1 M HEPES (final 10 mM), and 300 µL L-glutamine (final 1%); and
RPMI 1640 medium: 26 mL RPMI 1640 (same as above), 3 mL FBS (final 10%), 300 µL 100x Penicillin/Streptomycin (final 1x), 30 µL 2-ME (final 53 µM), 300 µL 1 M HEPES (final 10 mM), and 300 µL L-glutamine (final 1%).

The cells cultured as described above were washed 3 times with PBS, and 50 µL of 4% paraformaldehyde/PBS was added to each tube to fix the cells at room temperature for 10 minutes. The cells after fixation were washed 3 times with PBS, and 50 µL of 0.2% Triton X-100/PBS was added to each tube and allowed to react at room temperature for 10 minutes to permeate the cells.

The cells after permeation were washed 3 times with PBS, and blocked to prevent non-specific binding of the antibodies to Fc-receptors. The blocking was performed by adding, to each tube, 50 µL of 2% BSA/PBS solution containing 0.1 µL of TruStain FcX (anti-mouse CD16/32) Antibody (Clone 93 Isotype Rat IgG2a, λ (trade name), BioLegend, Cat. No. 101320) and incubated on ice for 10 minutes.

The supernatant was removed from each tube containing the cells, and 50 µL of 10% BSA/PBS was added to each tube and allowed to stand at room temperature for 1 hour to block the whole surface of the cells. Then, 25 µL of a mixture of the DNA barcode-labeled antibodies prepared in the above 1-1 or a mixture of the DNA barcodes was added to each tube, and allowed to contact with the cells at 4°C for 30 minutes. The cells were washed 3 times with PBS, and 50 µL of 4% paraformaldehyde/PBS was added to each tube and allowed to stand at room temperature for 10 minutes for post-fixation. The post-fixed cells were washed 3 times with PBS, and stored at 4°C with addition of 200 µL of PBS.

### 1-3 Joining PCR

### Preparation of hydrogel

Joining PCR was performed following a previous report (J. A. Weinstein et al., Cell, 2019, 178:229-241.e1-e10). As raw materials of a hydrogel, 4arm PEG and 2SH PEG were prepared. Since these two types of PEGs, when combined together, form a gel within about 2 minutes, 4arm PEG and 2SH PEG were separately prepared in ultrapure water in a 5 mL tube to a final concentration of 500 µg/µL. The required volume of each PEG was determined to be 20 µL per specimen. Specifically, at least 10 mg of powder PEG as a required amount was taken in a tube, and centrifuged at 5000 rpm at room temperature for 1 minute. Ultrapure water was added in an amount equivalent to the powder, and centrifuged at 5000 rpm at room temperature for 1 minute. The tube was mixed with inversion to allow the powder to be in contact with water, and centrifuged again. Inversion mixing and centrifugation were repeated until the solution became transparent. The liquid volume was measured with a pipette tip, and ultrapure water was added again so that the volume added was 2-fold of the initial water volume. The PEG solution was vortexed for homogeneous mixing, and centrifuged at 5000 rpm at room temperature for 1 minute.

### Design and synthesis of primers

The specimens prepared in the above 1-2 were washed twice with ultrapure water, and the PCR reaction solution as described later was added to each tube to perform joining PCR of the antibody barcodes in close proximity. Primers 1 and 2 serving as joining primers are indicated as Acryd-oligo 1 and Acryd-oligo 2, respectively, in Fig. 2-1 and Fig. 2-2, and were designed to each have a sequence that hybridizes to the 5' region of its paired primer. Acryd-oligo 1 had a sequence that hybridizes to the 3' region of a DNA barcode (target 1 oligo), and Acryd-oligo 2 had a sequence that hybridizes to the 3' region of a DNA barcode (target 2 oligo). Both primers also contained a random sequence (UEI) for distinguishing individual primer molecules. The joining primers were synthesized by a known technique, and modified with Acridite at the 5' end by a conventional method. Primers 3 and 4, as shown in Fig. 2-1, serve as amplification primers for amplifying the DNA barcodes that are joined together when at least two DNA barcode-labeled antibodies come in close proximity in cells. Primers 3 and 4 were designed to contain the 5' sequences of the DNA barcodes, and synthesized by a known technique. The UEI sequence and the primer sequences are as follows:
- UEI: NNNNNATNNNNN (SEQ ID NO: 6),
- Primer 1:
   5' Acryd-TATTCCCATGGCGCGCCANNNNNATNNNNNTTGAGGTGTCCTAAACTTACGC (SEQ ID NO: 7),
- Primer 2:
   5' Acryd-GGCGCGCCATGGGAATAATINNNNATTINNNNTGGAGTGGTCTCAACATATCGC (SEQ ID NO: 8),
- Primer 3:
   TCCCTACACGACGCTCTTCCGATCT (SEQ ID NO: 9), and
- Primer 4:
   GTTCAGACGTGTGCTCTTCCGATCT (SEQ ID NO: 10).

### Preparation of PCR reaction solution

The PCR reaction solution was prepared by combining the total volumes of solutions A and B prepared as below for each reaction. Solution A: 0.8 µL primer 1 (10 µM), 0.8 µL primer 2 (10 µM), 0.8 µL primer 3 (1 µM), 0.8 µL primer 4 (1 µM), 2 µL dNTPs (2 mM), 2.56 µL 4arm PEG (500 µg/µL, Laysan Bio, 4 ARM-PEG-ACRL-10K), 0.2 µL Q5 polymerase (trade name) (NEB), 4 µL Q5 buffer (x5, NEB), and 2.08 µL milli-Q water. Solution B: 1 µL BSA (10 mg/mL), 3.2 µL glycerol (50%), and 1.76 µL 2arm thiol PEG (500 µg/µL, Laysan Bio, SH-PEG-SH-3400). The solutions prepared as described above were combined on ice to prepare the PCR reaction solution. The PCR reaction solution at a volume of 20 µL was placed flat on the glass bead in each tube and allowed to stand at room temperature for 1 hour to solidify into a gel. The PCR reaction was performed through a program consisting of six steps: step 1: 95°C, 2 minutes; step 2: (95°C, 30 seconds and 68°C, 1 minute) x 10 cycles; step 3: (95°C, 30 seconds, 55°C, 30 seconds and 68°C, 1 minute) x 2 cycles; step 4: (95°C, 30 seconds, 60°C, 30 seconds and 68°C, 1 minute) x 16 cycles; step 5: 68°C, 1 minute; and step 6: 4°C, infinity. The samples after completion of the PCR reaction were stored at -20°C.

### Extraction of PCR products

The samples after PCR amplification were placed on ice, and 4 µL of PEG-dissolution mix was added flat to each sample tube and allowed to stand on ice for 2 hours to dissolve the gel. The composition of PEG-dissolution mix is as follows: 69.1 µL 1 N KOH (final 460 mM), 30 µL 0.5 M EDTA (final 100 mM), 6 µL 1 M DTT (final 40 mM), and 44.9 µL milli-Q water.

The samples dissolved as above were heated at 72°C for 5 minutes, and mixed 10 times by pipetting. Neutralization mix and Proteinase mix as described below were added to each sample tube at 4 µL and 11.1 µL, respectively. Neutralization mix: 400 µL 1 N HCl, and 600 µL 1 M Tris-HCl (pH 7.5). Proteinase mix: 12.4 µL 10% Tween20 (final 0.35 (v/v)%), 6.2 µL 20 mg/mL ProK (final 0.35 mg/mL), 3.6 µL 1 M Tris-HCl (pH 8) (final 10 mM), and 333 µL Milli-Q water. The samples were mixed by pipetting at each addition of the mixes. The samples were heated at 50°C for 25 minutes, and 55 µL of 10 mM Tris-HCl (pH 8) was added to each sample tube. After mixing by pipetting, 85 µL of each sample was transferred to a fresh PCR tube.

### 1-4 Next-generation sequencer analysis

### Preparation of libraries

The PCR products extracted as described above were purified and used to prepare sequencing libraries. Purification of the PCR products was performed using a commercially available purification kit (trade name: AMPure XP, Beckman Coulter, Cat. No. A63881) following the manufacturer's protocol. Briefly, AMPure XP stored at 4°C was brought back to room temperature and the magnetic beads were resuspended. A 0.65-fold volume of AMPure XP was added to each sample, and mixed by pipetting and allowed to stand at room temperature for 10 minutes. After spin down, the sample was allowed to stand on a magnet for 5 minutes, and the supernatant was removed. The sample on the magnet was washed twice with 70% ethanol, and dried for 3 minutes. The sample was removed from the magnet, and 35 µL of 10 mM Tris-HCl (pH 8) was added and mixed by pipetting and allowed to stand for 2 minutes. The sample was allowed to stand on the magnet for 5 minutes, and the supernatant was collected in a fresh PCR tube. The OD value was measured with a spectrometer (Bio Drop, BioDrop Ltd) to examine the quality and yield of the collected DNA.

### Tagging PCR

Tagging PCR of the DNA purified as above was performed for analysis with a next-generation sequencer. The PCR reaction was performed using a DNA polymerase (HiFi mix, Nippon Genetics) through a program consisting of three steps: step 1: 98°C, 30 seconds; step 2: (98°C, 10 seconds and 65°C, 75 seconds) × 12 cycles; and step 3: 65°C, 5 minutes. The PCR reaction solution in a total volume of 50 µL was prepared to have the following composition: 2.5 µL PE1.0 (10 µM), 2.5 µL iPCRtag_X_L 2 (10 µM), 1.65 µL primer 5 (100 µM), 1.65 µL primer 6 (100 µM), 25 µL HiFi mix (2x), 1 µL sample, and 15.7 µL milli-Q water. The sequences of the primers are shown below. Primers 5 and 6 were designed to be complementary sequences to the 5' sequences of primers 1 and 2, and synthesized by a known technique.
- PE1.0: 
AATGATACGGCGACCACCGAGATCTACACTCTTTCCCTACACGACGCTCTTCC
GATCT (SEQ ID NO: 11)
- iPCRtag_X_L2: 
CAAGCAGAAGACGGCATACGAGATNNNNNNGTGACTGGAGTTCAGACGTGT
GCTCTTCCGATCT (SEQ ID NO: 12)
- Primer 5:
   TGGCGCGCCATGGGAATAA (SEQ ID NO: 13)
- Primer 6:
   TTATTCCCATGGCGCGCCA (SEQ ID NO: 14)

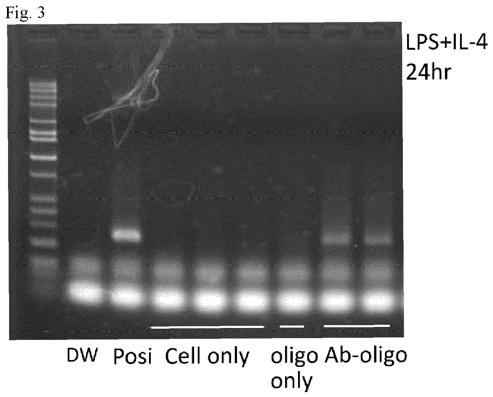

The samples amplified by tagging PCR were run on a 2% agarose gel (TAE) by electrophoresis. The results are shown in Fig. 3. Bands of 267 bp were detected only in the samples using the DNA barcode-labeled antibodies, indicating the formation of the joined DNA barcodes. The bands of 267 bp size were excised, and the PCR products were extracted using a commercially available kit (trade name: Monarch DNA Gel Extraction Kit, NEW ENGLAND BioLabs) following the manufacturer's protocol. The success or failure of the joining PCR was determined by Sanger sequencing of the extracted PCR products. The PCR products extracted as above were cloned using a commercially available kit (Zero Blunt PCR Cloning Kit, Thermo Fisher) and then subjected to Sanger sequencing to confirm the antibody barcode sequences.

The next-generation sequence analysis was commissioned to Macrogen Japan Corp. The sequence data were obtained with a next-generation sequencer of Illumina. BCL files were created using the primary analysis software RTA, and converted into FASTQ using bcl2fastq2-v2.20.0. The structures of the reads are shown in Figs. 2-1 and 2-2.

### 2. Results

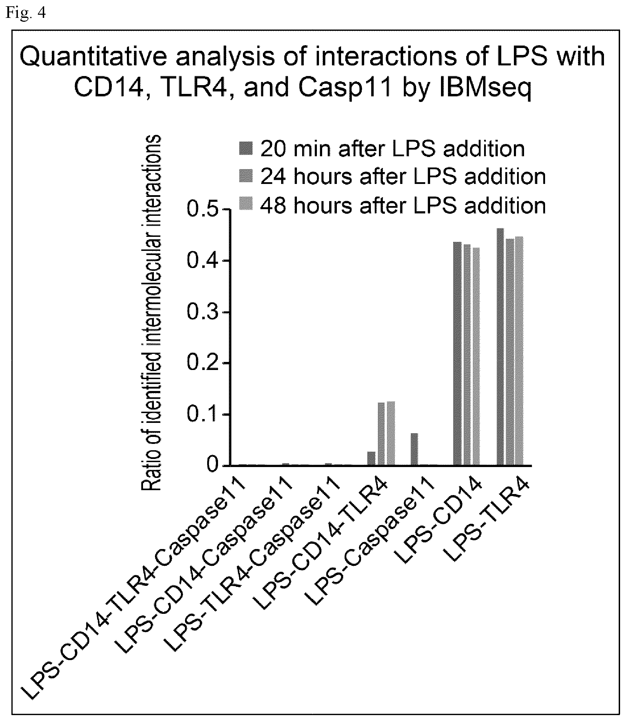

It was demonstrated that the method of the present invention can be used to detect multifactorial interactions of LPS with CD14, TLR4 and/or Caspase-11 in B cells isolated from the spleen. Fig. 4 shows the results of next-generation sequence analysis. After LPS administration, temporary binding of LPS and Caspase-11, and prolonged binding of LPS to CD14 and TLR4 were observed in B cells collected from the mouse spleen. In particular, the sequence analysis of the PCR products containing the joined DNA barcodes amplified from the cell samples 20 minutes and 24 and 48 hours after LPS administration revealed that LPS and Caspase-11 bind together immediately after LPS administration and remain bonded together until 20 minutes after LPS administration, but the binding dissociates over time (Fig. 4). Binding of LPS and CD14 and binding of LPS and TLR4 were observed through the entire analysis period, whereas trimer formation of LPS with CD14 and TLR4 was promoted over time (Fig. 4).

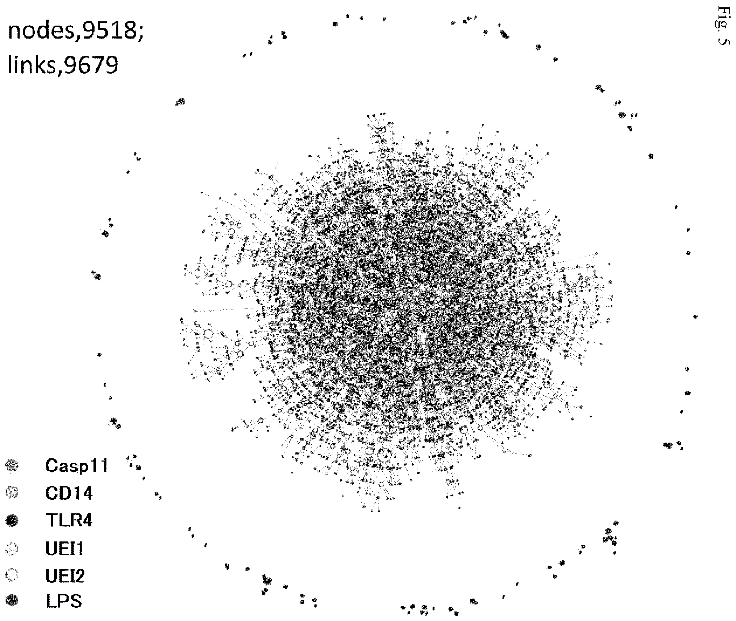

Fig. 5 shows a network diagram showing interactions among LPS, Caspase-11, CD14 and TLR4 after LPS administration. The dot size is proportional to the number of sequencing reads. Only the interactions in which binding between multiple factors was observed were extracted, and plotted in a network diagram, which indicates that a single factor interacts with multiple factors (Fig. 5).

### Example 2: Identification of multifactorial interactions by LPS stimulation

### 1. Materials and methods

### 1-1 Production of DNA Barcode-conjugated antibodies

### Preparation of biotinylated DNA barcodes

DNA barcodes having antibody barcode sequences, UMI sequences and adapter sequences for sequencing were designed for factors known to interact with each other after LPS stimulation (phosphorylated RNA polymerase II-CTD (Pol2S2P), Lamin B1, c-Myc, acetylated histone (H3K27ac), and p300) and IgG control, and the DNA barcodes were synthesized by a known technique in the same manner as in Example 1. The 5' ends of the DNA barcodes were biotinylated by a conventional method, and the DNA barcodes were diluted in Tris-EDTA buffer to 100 µM, heated at 55°C for 10 minutes, and centrifuged at 18000xg at room temperature for 5 minutes. The concentration in the supernatant was checked and adjusted again, if necessary, to 100 µM. The sequences of the DNA barcodes are as follows. The antibody barcode sequences are underlined.
- target 1 oligo (5biotin)_BC1 (DNA barcode for anti-Pol2S2P antibody): 
GACGCTCTTCCGATCTNNNNNNNWWSNNNWWWNNNNSACCGTGATGCGTA
AGTTTAGGACACCTCA
(SEQ ID NO: 15)
- target 1 oligo (5biotin)_BC2 (DNA barcode for anti-Lamin B1 antibody): 
GACGCTCTTCCGATCTNNNNNNNWWSNNNWWWNNNNSACACATCGGCGTA
AGTTTAGGACACCTCA
(SEQ ID NO: 16)
- target 1 oligo (5biotin)_BC3 (DNA barcode for anti-cMYC antibody): 
GACGCTCTTCCGATCTNNNNNNNWWSNNNWWWNNNNSACGCCTAAGCGTA
AGTTTAGGACACCTCA
(SEQ ID NO: 17)
- target 1 oligo (5biotin)_BC4 (DNA barcode for anti-H3K27ac antibody): 
GACGCTCTTCCGATCTNNNNNNNWWSNNNWWWNNNNSACTGGTCAGCGTA
AGTTTAGGACACCTCA
(SEQ ID NO: 18)
- target 1 oligo (5biotin)_BC5 (DNA barcode for anti-p300 antibody): 
GACGCTCTTCCGATCTNNNNNNNWWSNNNWWWNNNNSACTCAAGTGCGTA
AGTTTAGGACACCTCA
(SEQ ID NO: 19)
- target 2 oligo (5biotin)_BC6 (DNA barcode for anti-Pol2S2P antibody): 
TGTGCTCTTCCGATCTNNNNNNNWWSNNNWWWNNNNSACCTCTACGCGAT
ATGTTGAGACCACTCC
(SEQ ID NO: 20)
- target 2 oligo (5biotin)_BC7 (DNA barcode for anti-Lamin B1 antibody): 
TGTGCTCTTCCGATCTNNNNNNNWWSNNNWWWNNNNSACGCTACCGCGAT
ATGTTGAGACCACTCC
(SEQ ID NO: 21)
- target 2 oligo (5biotin)_BC8 (DNA barcode for anti-cMYC antibody): 
TGTGCTCTTCCGATCTNNNNNNNWWSNNNWWWNNNNSACGCTCATGCGAT
ATGTTGAGACCACTCC
(SEQ ID NO: 22)
- target 2 oligo (5biotin)_BC9 (DNA barcode for anti-H3K27ac antibody): 
TGTGCTCTTCCGATCTNNNNNNNNWWSNNNWWWNNNNSACCTTTGGCGAT
ATGTTGAGACCACTCC
(SEQ ID NO: 23)
- target 2 oligo (5biotin)_BC10 (DNA barcode for anti-p300 antibody): 
TGTGCTCTTCCGATCTNNNNNNNWWSNNNWWWNNNNSACATCGTGGCGAT
ATGTTGAGACCACTCC
(SEQ ID NO: 24)
- target 1 oligo (5biotin)_BC11 (DNA barcode for IgG): 
GACGCTCTTCCGATCTNNNNNNNWWSNNNWWWNNNNSACCACTGTGCGTA
AGTTTAGGACACCTCA
(SEQ ID NO: 25)
- target 2 oligo (5biotin)_BC12 (DNA barcode for IgG):

TGTGCTCTTCCGATCTNNNNNNNWWSNNNWWWNNNNSACTAGTTGGCGAT
ATGTTGAGACCACTCC
(SEQ ID NO: 26)

### Preparation of antibody-streptavidin complexes

The following antibodies were labeled with streptavidin in the same manner as in Example 1: anti-Phospho-RNA pol II CTD (Ser2) antibody (Pol2S2P, rabbit monoclonal antibody, clone 7H23L1, Thermo-Fisher, Cat. No. 703108), anti-Lamin B1 antibody (mouse monoclonal antibody, clone L-5, Thermo-Fisher, Cat. No. 33-2000), anti-c-Myc antibody (mouse monoclonal antibody, clone 9E10, Thermo-Fisher, Cat. No. 13-2500), anti-acetylated histone antibody (H3K27ac, mouse monoclonal antibody, clone GT261, Thermo-Fisher, Cat. No. MA5-31555), and anti-p300 antibody (mouse monoclonal antibody, clone RW128, Thermo-Fisher, Cat. No. MA1-16608), and IgG (mouse, BioLegend, Cat. No. 400101).

### Preparation of DNA barcode-labeled antibodies

The streptavidin labeled-antibodies were allowed to bind to the corresponding biotinylated DNA barcodes to form conjugates in the same manner as in Example 1. The combinations of the antibodies and the DNA barcodes are as follows: anti-Pol2S2P antibody-target 1 oligo (5biotin)_BC1 (SEQ ID NO: 15), anti-Lamin B1 antibody-target 1 oligo (5biotin)_BC2 (SEQ ID NO: 16), anti-cMYC antibody-target 1 oligo (5biotin)_BC3 (SEQ ID NO: 17), anti-H3K27ac antibody-target 1 oligo (5biotin)_BC4 (SEQ ID NO: 18), anti-p300 antibody-target 1 oligo (5biotin)_BC5 (SEQ ID NO: 19), anti-Pol2S2P antibody-target 2 oligo (5biotin)_BC6 (SEQ ID NO: 20), anti-Lamin B1 antibody-target 2 oligo (5biotin)_BC7 (SEQ ID NO: 21), anti-cMYC antibody-target 2 oligo (5biotin)_BC8 (SEQ ID NO: 22), anti-H3K27ac antibody-target 2 oligo (5biotin)_BC9 (SEQ ID NO: 23), anti-p300 antibody-target 2 oligo (5biotin)_BC10 (SEQ ID NO: 24), normal IgG-target 1 oligo (5biotin)_BC11 (SEQ ID NO: 25), and normal IgG-target 2 oligo (5biotin)_BC12 (SEQ ID NO: 26). The solvent of the solutions containing one of the DNA barcode-labeled antibodies after the reaction was exchanged with PBS by a conventional method, and the total volume was adjusted to 30 µL with PBS.

The DNA barcode-labeled antibodies prepared as above were run by electrophoresis on a 4% TAE gel for 10 minutes. The gel was then treated with ethidium bromide for 10 minutes to visualize the bands to examine the conjugation of the DNA barcodes. As controls, 50 pmol of the DNA barcodes were run, and the relative band intensities were determined for all bands appeared on the lanes of the DNA barcode-labeled antibody samples by image analysis. The amounts of the DNA barcodes in the prepared DNA barcode-labeled antibodies were estimated. Samples for which successful conjugation of the DNA barcodes was confirmed were stored at 4°C after addition of 50 mg/mL BSA and 1% sodium azide at final concentrations of 1 µg/µL and 0.05%, respectively.

### 1-2 Preparation of specimens

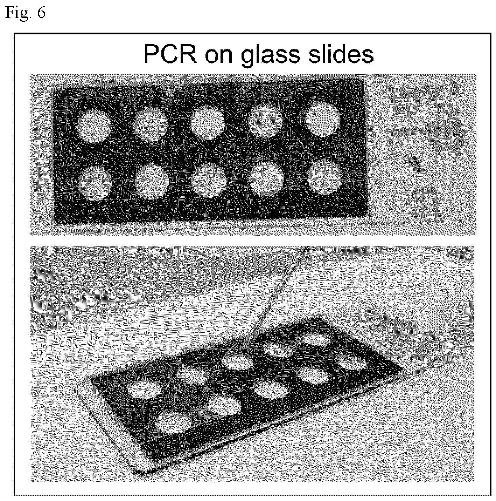

B cells collected from mouse spleen were suspended in serum-free medium. The cells were seeded on each well of fluororesin-coated glass slides (super hydrophobic printed glass slides, 10 wells, 6 mm diameter, Matsunami Glass Ind., Ltd., Cat. No. TF1006, see Fig. 6) with an adjusted density of 5000 cells/30 µL per circle. The cells were cultured in 5% CO₂ at 37°C for 30 minutes, and the culture supernatant was removed. Then, 1 mg/mL LPS and 2.5 µg/mL IL-4 were diluted in RPMI 1640 medium to final concentrations of 50 µg/mL and 2.5 ng/mL, respectively, and added to each well at 50 µL each. The cells were cultured in 5% CO₂ at 37°C for 24 hours. IL-4 was used as stimulation for B-cell activation.

The cells stimulated with LPS as described above were washed 3 times with PBS, and 50 µL of 4% paraformaldehyde/PBS was added to each well to fix the cells at room temperature for 10 minutes. Ater fixation of the cells, 30 µL of 0.2% Triton X-100/PBS was added to each well and allowed to react at room temperature for 10 minutes to permeate the cells.

The cells after permeation were washed 3 times with PBS, and blocked to prevent non-specific binding of the antibodies to Fc-receptors. The blocking was performed by adding, to each well, 30 µL of 10% BSA/PBS solution containing 2%TruStain FcX (anti-mouse CD16/32) Antibody (Clone 93 Isotype Rat IgG2a, λ (trade name), BioLegend, Cat. No. 101320) and incubated at 4°C overnight.

Then, 25 µL of a mixture of the DNA barcode-labeled antibodies prepared in the above 1-1 or a mixture of the DNA barcodes was added to each well, and allowed to contact with the cells at 4°C for 30 minutes. The cells were washed 7 times with PBS containing 0.1% Tween20, and 40 µL of 4% paraformaldehyde/PBS was added to each well and allowed to stand at room temperature for 10 minutes for post-fixation. The post-fixed cells were washed 3 times with PBS containing 0.1% Tween20, and stored at 4°C with addition of 50 µL of PBS.

### 1-3 Joining PCR

Hydrogel was prepared and joining PCR was performed in the same manner as in Example 1. The PCR reaction solution, at a volume of 20 µL, prepared in the same manner as in Example 1 was added to the specimens prepared in the above 1-2 and allowed to solidify into a gel, and frame-seal slide chambers and plastic covers (BIO-RAD, Cat. No. SLF0201) were placed on the glass slides (see Fig. 6). The joining PCR of the antibody barcodes that come in close proximity was performed using Mastercycler nexus flat (trade-name) (Eppendorf) PCR cycler. Primers used were the joining primers as set forth in SEQ ID NOs: 7 and 8, and the amplification primers as set forth in SEQ ID NOs: 9 and 10. The PCR reaction was performed through a program consisting of six steps: step 1: 95°C, 2 minutes; step 2: (95°C, 30 seconds and 68°C, 1 minute) × 10 cycles; step 3: (95°C, 30 seconds, 55°C, 30 seconds and 68°C, 1 minute) × 2 cycles; step 4: (95°C, 30 seconds, 60°C, 30 seconds and 68°C, 1 minute) × 16 cycles; step 5: 68°C, 1 minute; and step 6: 4°C, infinity. The samples after completion of the PCR reaction were stored at -20°C.

### Extraction of PCR products

The samples after PCR amplification were placed on ice, and 4 µL of PEG-dissolution mix was added flat to each chamber and allowed to stand on ice for 2 hours to dissolve the gel. The dissolved samples were heated at 72°C for 5 minutes, and mixed 10 times by pipetting. Neutralization mix and Proteinase mix were added to each chamber at 4 µL and 11.1 µL, respectively. The samples were mixed by pipetting at each addition of the mixes. The samples were heated at 50°C for 25 minutes, and 55 µL of 10 mM Tris-HCl (pH 8) was added to each chamber. After mixing by pipetting, 85 µL of each sample was transferred to a fresh PCR tube.

### 1-4 Next-generation sequencer analysis

The PCR products extracted as described above were purified in the same manner as in Example 1, and used to prepare sequencing libraries. Tagging PCR and electrophoresis on a 2% TAE gel were performed in the same manner as in Example 1. The PCR products were extracted using a commercially available kit (trade name: Monarch DNA Gel Extraction Kit, NEW ENGLAND BioLabs) following the manufacturer's protocol. The extracted PCR products were sequenced using HiSeq system of Illumina in the same manner as in Example 1.

### 2. Results

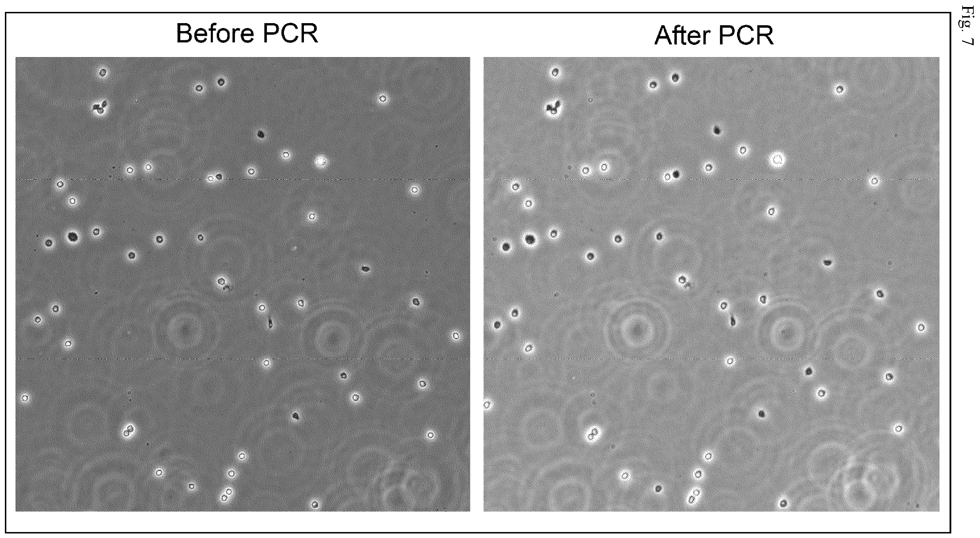

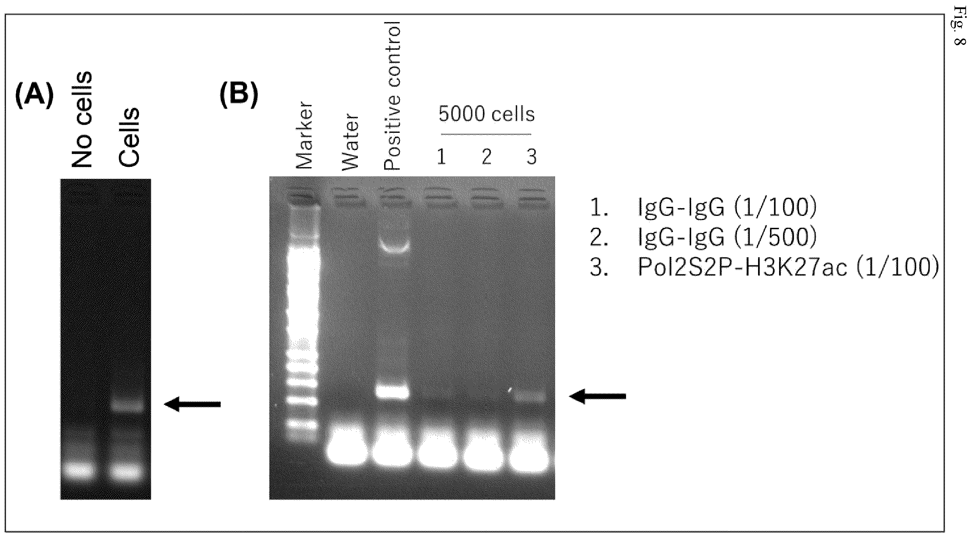

The method of the present invention could identify that multifactorial interactions occur among phosphorylated RNA polymerase II-CTD (Pol2S2P), Lamin B1, c-Myc, acetylated histone (H3K27ac), and p300 after LPS stimulation in B cells isolated from the spleen. The B cells seeded on the glass slides were examined under a microscope before and after the PCR reaction, and showed that the morphology of the cells were maintained (Fig. 7), indicating that the spatial information was maintained during joining PCR. The gel electrophoresis after the PCR reaction showed that bands of PCR products were not detected in the absence of cells or when IgG as control was used, confirming that the method of the present invention is able to specifically detect multifactorial interactions in a biological sample (Fig. 8).

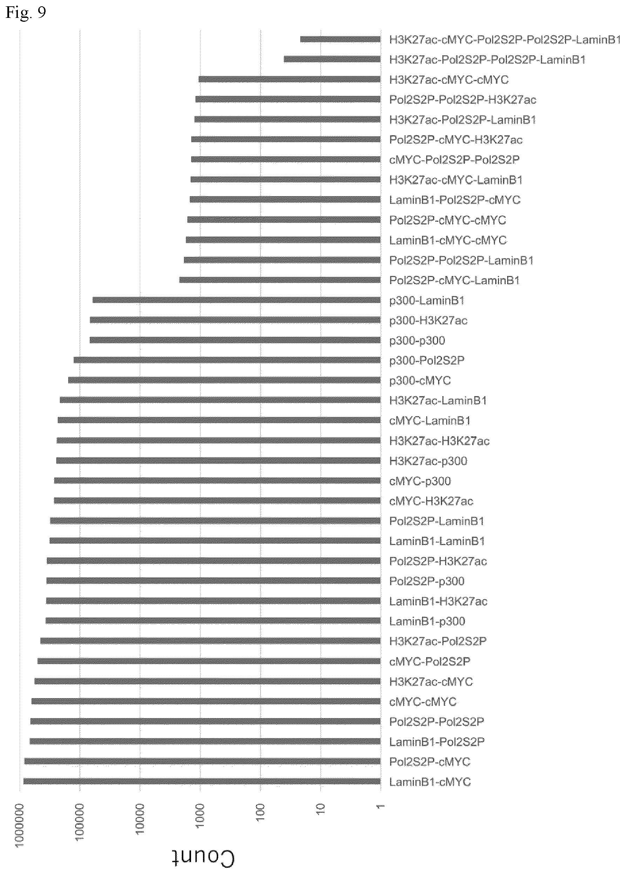

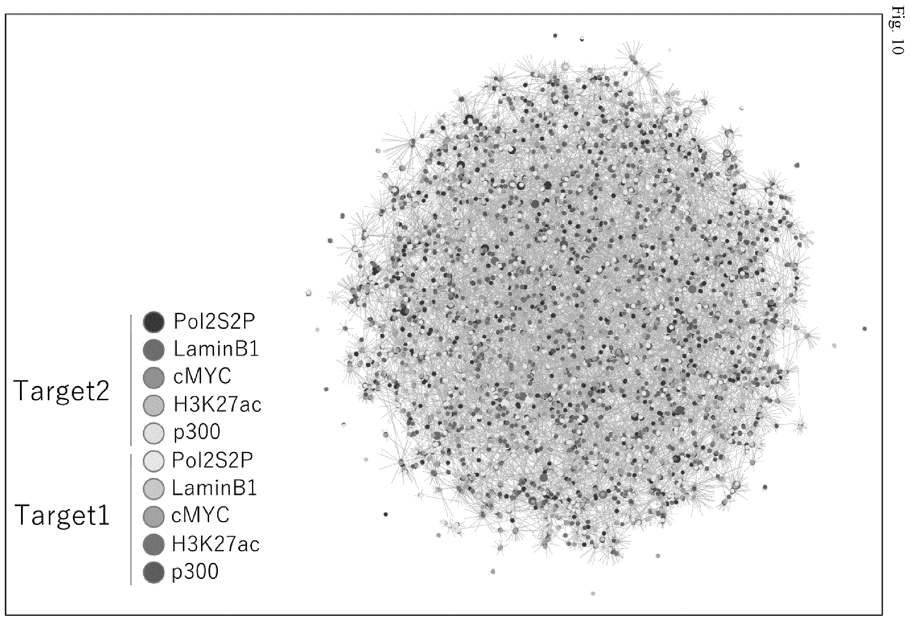

Fig. 9 shows the results of next-generation sequence analysis. In LPS-stimulated B cells from mouse spleen, interactions between any two of the factors Pol2S2P, Lamin B1, c-Myc, H3K27ac, and p300 were detected, and also interactions among three of the factors, and four of the factors were detected. Only the interactions in which binding between multiple factors was observed were extracted, and plotted in a network diagram, which indicates that a single factor interacts with multiple factors (Fig. 10). These results revealed that, the method of the present invention is able to perform quantitative analysis of intermolecular interactions by targeting multiple factors.

### Example 3: Identification of multifactorial interactions by LPS stimulation

### 1. Materials and methods

### 1-1 Production of DNA barcode-conjugated antibodies

### Preparation of biotinylated DNA barcodes

DNA barcodes having antibody barcode sequences, UMI sequences and adapter sequences for sequencing were designed for factors known to interact with each other after LPS stimulation and IgG control, and the DNA barcodes were synthesized by a known technique in the same manner as in Example 2. The 5' ends of the DNA barcodes were biotinylated by a conventional method, and the DNA barcodes were diluted in Tris-EDTA buffer to 100 µM, heated at 55°C for 10 minutes, and centrifuged at 18000xg at room temperature for 5 minutes. The concentration in the supernatant was checked and adjusted again, if necessary, to 100 µM. The analytical target factors were as follows: phosphorylated RNA polymerase II-CTD (Ser2) (Pol2S2P), Lamin B1, c-Myc, acetylated histone (H3K27ac), p300, histone deacetylase 1 (HDAC1), CPSF73, histone H3, phosphorylated RNA polymerase II-CTD (Ser5) (Pol2S5P), methylated histone (H3K4Me1), methylated histone (H3K27Me3), methylated histone (H3K4Me3), Rpb1 NTD (Pol2NTD), CTCF (D31H2), XPB (2C6), and MED26 (D4B 1X). The sequences of the DNA barcodes are shown below. The antibody barcode sequences are underlined. The same DNA barcodes as those in Example 2 were used for phosphorylated RNA polymerase II-CTD (Ser2) (Pol2S2P), Lamin B1, c-Myc, acetylated histone (H3K27ac), and p300.
- target 1 oligo (5biotin)_BC8 (DNA barcode for anti-HDAC1 antibody): 
GACGCTCTTCCGATCTNNNNNNNWWSNNNWWWNNNNSACTCAAGTGCGTA
AGTTTAGGACACCTCA
(SEQ ID NO: 27)
- target 1 oligo (5biotin)_BC9 (DNA barcode for anti-CPSF73 antibody): 
GACGCTCTTCCGATCTNNNNNNNWWSNNNWWWNNNNSACCTGATCGCGTA
AGTTTAGGACACCTCA
(SEQ ID NO: 28)
- target 1 oligo (5biotin)_BC10 (DNA barcode for anti-Histone H3 antibody): 
GACGCTCTTCCGATCTNNNNNNNWWSNNNWWWNNNNSACAAGCTAGCGTA
AGTTTAGGACACCTCA
(SEQ ID NO: 29)
- target 1 oligo (5biotin)_BC7 (DNA barcode for anti-Pol2S5P antibody): 
GACGCTCTTCCGATCTNNNNNNNWWSNNNWWWNNNNSACGATCTGGCGTA
AGTTTAGGACACCTCA
(SEQ ID NO: 30)
- target 1 oligo (5biotin)_BC11 (DNA barcode for anti-H3K4Me1 antibody): 
GACGCTCTTCCGATCTNNNNNNNWWSNNNWWWNNNNSACGTAGCCGCGTA
AGTTTAGGACACCTCA
(SEQ ID NO: 31)
- target 1 oligo (5biotin)_BC12 (DNA barcode for anti-H3K27Me3 antibody): 
GACGCTCTTCCGATCTNNNNNNNWWSNNNWWWNNNNSACTACAAGGCGTA
AGTTTAGGACACCTCA
(SEQ ID NO: 32)
- target 1 oligo (5biotin)_BC13 (DNA barcode for anti-H3K4Me3 antibody): 
GACGCTCTTCCGATCTNNNNNNNWWSNNNWWWNNNNSACTGTTGACTGCG
TAAGTTTAGGACACCTCA
(SEQ ID NO: 33)
- target 1 oligo (5biotin)_BC14 (DNA barcode for anti-Pol2NTD antibody): 
GACGCTCTTCCGATCTNNNNNNNWWSNNNWWWNNNNSACACGGAACTGC
GTAAGTTTAGGACACCTCA
(SEQ ID NO: 34)
- target 1 oligo (5biotin)_BC15 (DNA barcode for anti-CTCF antibody): 
GACGCTCTTCCGATCTNNNNNNNWWSNNNWWWNNNNSACTCTGACATGCG
TAAGTTTAGGACACCTCA
(SEQ ID NO: 35)
- target 1 oligo (5biotin)_BC16 (DNA barcode for anti-XPB antibody): 
GACGCTCTTCCGATCTNNNNNNNWWSNNNWWWNNNNSACGGACGGGCGT
AAGTTTAGGACACCTCA
(SEQ ID NO: 36)
- target 1 oligo (5biotin)_BC18 (DNA barcode for anti-MED26 antibody): 
GACGCTCTTCCGATCTNNNNNNNWWSNNNWWWNNNNSACGTGCGGACGC
GTAAGTTTAGGACACCTCA
(SEQ ID NO: 37)
- target 2 oligo (5biotin)_BC33 (DNA barcode for anti-HDAC1 antibody): 
TGTGCTCTTCCGATCTNNNNNNNWWSNNNWWWNNNNSACCGCCTGGCGAT
ATGTTGAGACCACTCC
(SEQ ID NO: 38)
- target 2 oligo (5biotin)_BC41 (DNA barcode for anti-CPSF73 antibody): 
TGTGCTCTTCCGATCTNNNNNNNWWSNNNWWWNNNNSACGTCGTCGCGAT
ATGTTGAGACCACTCC
(SEQ ID NO: 39)
- target 2 oligo (5biotin)_BC35 (DNA barcode for anti-Histone H3 antibody): 
TGTGCTCTTCCGATCTNNNNNNNWWSNNNWWWNNNNSACAAAATGGCGAT
ATGTTGAGACCACTCC
(SEQ ID NO: 40)
- target 2 oligo (5biotin)_BC32 (DNA barcode for anti-Pol2S5P antibody): 
TGTGCTCTTCCGATCTNNNNNNNWWSNNNWWWNNNNSACTGAGTGGCGAT
ATGTTGAGACCACTCC
(SEQ ID NO: 41)
- target 2 oligo (5biotin)_BC36 (DNA barcode for anti-H3K4Me1 antibody): 
TGTGCTCTTCCGATCTNNNNNNNWWSNNNWWWNNNNSACTGTTGGGCGAT
ATGTTGAGACCACTCC
(SEQ ID NO: 42)
- target 2 oligo (5biotin)_BC42 (DNA barcode for anti-H3K27Me3 antibody): 
TGTGCTCTTCCGATCTNNNNNNNWWSNNNWWWNNNNSACCGATTAGCGAT
ATGTTGAGACCACTCC
(SEQ ID NO: 43)
- target 2 oligo (5biotin)_BC30 (DNA barcode for anti-H3K4Me3 antibody): 
TGTGCTCTTCCGATCTNNNNNNNWWSNNNWWWNNNNSACCCGGTGGCGAT
ATGTTGAGACCACTCC
(SEQ ID NO: 44)
- target 2 oligo (5biotin)_BC37 (DNA barcode for anti-Pol2NTD antibody): 
TGTGCTCTTCCGATCTNNNNNNNWWSNNNWWWNNNNSACATTCCGGCGAT
ATGTTGAGACCACTCC
(SEQ ID NO: 45)
- target 2 oligo (5biotin)_BC38 (DNA barcode for anti-CTCF antibody): 
TGTGCTCTTCCGATCTNNNNNNNWWSNNNWWWNNNNSACAGCTAGGCGAT
ATGTTGAGACCACTCC
(SEQ ID NO: 46)
- target 2 oligo (5biotin)_BC39 (DNA barcode for anti-XPB antibody): 
TGTGCTCTTCCGATCTNNNNNNNWWSNNNWWWNNNNSACGTATAGGCGAT
ATGTTGAGACCACTCC
(SEQ ID NO: 47)
- target 2 oligo (5biotin)_BC40 (DNA barcode for anti-MED26 antibody):

TGTGCTCTTCCGATCTNNNNNNNWWSNNNWWWNNNNSACTGGATCACGCG
ATATGTTGAGACCACTCC
(SEQ ID NO: 48)

### Preparation of antibody-streptavidin complexes

The following monoclonal antibodies were labeled with streptavidin in the same manner as in Example 1: anti-HDAC1antibody (mouse antibody, clone 10E2, Thermo-Fisher, Cat. No. MA5-18071), anti-CPSF73 antibody (mouse antibody, clone 6E6, Novus Biologicals, Cat. No. H00051692-M01), anti-histone H3 antibody (rabbit antibody, clone EPR16987, abcam, Cat. No. ab238971), anti-Pol2S5P antibody (mouse antibody, clone 4H8, Thermo-Fisher, Cat. No. MA1-46093), anti-H3K4Me1 antibody (rabbit antibody, clone ERP16597, abcam, Cat. No. ab239402), anti-H3K27Me3 antibody (rabbit antibody, clone EPR18607, abcam, Cat. No. ab222481), anti-H3K4Me3 antibody (mouse antibody, clone CMA304, Merck, Cat. No. 05-1339), anti-Pol2NTD antibody (rabbit antibody, Cell Signaling Technology, Cat. No. 14958), anti-CTCF (D31H2) antibody (rabbit antibody, Cell Signaling Technology, Cat. No. 3418), anti-XPB (2C6) antibody (mouse antibody, Cell Signaling Technology, Cat. No. 8746), anti-MED26 (D4B1X) antibody (rabbit antibody, Cell Signaling Technology, Cat. No. 14950), and IgG (mouse antibody, BioLegend, Cat. No. 400101). The same antibodies as those in Example 2 were used for phosphorylated RNA polymerase II-CTD (Ser2) (Pol2S2P), Lamin B1, c-Myc, acetylated histone (H3K27ac), and p300.

### Preparation of DNA barcode-labeled antibodies

The streptavidin labeled-antibodies were allowed to bind to the corresponding biotinylated DNA barcodes to form conjugates in the same manner as in Example 1. The combinations of the antibodies and the DNA barcodes are as follows: anti-HDAC1 antibody-target 1 oligo (5biotin)_BC8 (SEQ ID NO: 27), anti-CPSF73 antibody-target 1 oligo (5biotin)_BC9 (SEQ ID NO: 28), anti-Histone H3 antibody-target 1 oligo (5biotin)_BC10 (SEQ ID NO: 29), anti-Pol2S5P antibody-target 1 oligo (5biotin)_BC7 (SEQ ID NO: 30), anti-H3K4Me1 antibody-target 1 oligo (5biotin)_BC11 (SEQ ID NO: 31), anti-H3K27Me3 antibody-target 1 oligo (5biotin)_BC12 (SEQ ID NO: 32), anti-H3K4Me3 antibody-target 1 oligo (5biotin)_BC13 (SEQ ID NO: 33), anti-Pol2NTD antibody-target 1 oligo (5biotin)_BC14 (SEQ ID NO: 34), anti-CTCF antibody-target 1 oligo (5biotin)_BC15 (SEQ ID NO: 35), anti-XPB antibody-target 1 oligo (5biotin)_BC16 (SEQ ID NO: 36), anti-MED26 antibody-target 1 oligo (5biotin)_BC18 (SEQ ID NO: 37), anti-HDAC1 antibody-target 2 oligo (5biotin)_BC33 (SEQ ID NO: 38), anti-CPSF73 antibody-target 2 oligo (5biotin)_BC41 (SEQ ID NO: 39), anti-Histone H3 antibody-target 2 oligo (5biotin)_BC35 (SEQ ID NO: 40), anti-Pol2S5P antibody-target 2 oligo (5biotin)_BC32 (SEQ ID NO: 41), anti-H3K4Me1 antibody-target 2 oligo (5biotin)_BC36 (SEQ ID NO: 42), anti-H3K27Me3 antibody-target 2 oligo (5biotin)_BC42 (SEQ ID NO: 43), anti-H3K4Me3 antibody-target 2 oligo (5biotin)_BC30 (SEQ ID NO: 44), anti-Pol2NTD antibody-target 2 oligo (5biotin)_BC37 (SEQ ID NO: 45), anti-CTCF antibody-target 2 oligo (5biotin)_BC38 (SEQ ID NO: 46), anti-XPB antibody-target 2 oligo (5biotin)_BC39 (SEQ ID NO: 47), and anti-MED26 antibody-target 2 oligo (5biotin)_BC40 (SEQ ID NO: 48). For phosphorylated RNA polymerase II-CTD (Ser2) (Pol2S2P), Lamin B1, c-Myc, acetylated histone (H3K27ac), and p300, the same combinations as those in Example 2 were used to create conjugates. The solvent of the solutions containing one of the DNA barcode-labeled antibodies after the reaction was exchanged with PBS by a conventional method, and the total volume was adjusted to 30 µL with PBS.

The DNA barcode-labeled antibodies prepared as above were run by electrophoresis on a 4% TAE gel for 10 minutes. The gel was then treated with ethidium bromide for 10 minutes to visualize the bands to examine the conjugation of the DNA barcodes. As controls, 50 pmol of the DNA barcodes were run, and the relative band intensities were determined for all bands appeared on the lanes of the DNA barcode-labeled antibody samples by image analysis. The amounts of the DNA barcodes in the prepared DNA barcode-labeled antibodies were estimated. Samples for which successful conjugation of the DNA barcodes was confirmed were stored at 4°C after addition of 50 mg/mL BSA and 1% sodium azide at final concentrations of 1 µg/µL and 0.05%, respectively.

### 1-2 Preparation of specimens

Specimens were prepared in the same manner as in Example 2. Briefly, B cells collected from mouse spleen were suspended in serum-free medium. The cells were seeded on each well of fluororesin-coated glass slides (super hydrophobic printed glass slides, 10 wells, 6 mm diameter, Matsunami Glass Ind., Ltd., Cat. No. TF1006, see Fig. 6) with an adjusted density of 5000 cells/30 µL per circle. The cells were cultured in 5% CO₂ at 37°C for 30 minutes, and the culture supernatant was removed. Then, 1 mg/mL LPS and 2.5 µg/mL IL-4 were diluted in RPMI 1640 medium to final concentrations of 50 µg/mL and 2.5 ng/mL, respectively, and added to each well at 50 µL each. The cells were cultured in 5% CO₂ at 37°C for 24 hours. IL-4 was used as stimulation for B-cell activation.

The cells stimulated with LPS as described above were washed 3 times with PBS, and 50 µL of 4% paraformaldehyde/PBS was added to each well to fix the cells at room temperature for 10 minutes. Ater fixation of the cells, 30 µL of 0.2% Triton X-100/PBS was added to each well and allowed to react at room temperature for 10 minutes to permeate the cells.

The cells after permeation were blocked in the same manner as in Example 2. Then, 25 µL of a mixture of the DNA barcode-labeled antibodies prepared in the above 1-1 or a mixture of the DNA barcodes was added to each well, and allowed to contact with the cells at 4°C for 30 minutes. The cells were washed 7 times with PBS containing 0.1% Tween20, and 40 µL of 4% paraformaldehyde/PBS was added to each well and allowed to stand at room temperature for 10 minutes for post-fixation. The post-fixed cells were washed 3 times with PBS containing 0.1% Tween20, and stored at 4°C with addition of 50 µL of PBS.

### 1-3 Joining PCR and next-generation sequencer analysis

Joining PCR was performed in the same manner as in Example 2. The PCR products were extracted and purified to prepare libraries for sequencing. Tagging PCR was performed and the PCR products were extracted. The extracted PCR products were sequenced using HiSeq system of Illumina. Low quality reads were trimmed off from the obtained reads using fastqc, and UMI1, UMI2, UEI1, UEI2, and the antibody barcodes were detected using umi_tools (https://umi-tools.readthedocs.io/en/latest/). UMI represents a sequence for distinguishing individual antibody molecules, and UEI represents a sequence for distinguishing individual primer molecules. Assessment of the antibody barcodes was performed by using an awk code. The reads containing proper antibody barcodes were collected, and analyzed using the R language extensions, igraph, data.table. dplyr, tidyverse, and ggplot2.

### 2. Results

The results are shown in Fig. 11. Fig. 11A shows the analytical results of the number of bindings of the anti-acetylated histone (H3K27ac) antibody molecules per UEI over time. The vertical axis indicates the UEI counts, the horizontal axis indicates the number of the anti-H3K27ac antibody molecules bound to a single UEI. It was demonstrated that the number of acetylated histone (H3K27ac) molecules bound to a single UEI increases over time after LPS stimulation (Fig. 11A). These results indicate that the number of bindings of the anti-H3K27ac antibody molecules per UEI increases by activation of B cells. Fig. 11B shows the results of analysis of the concentration of networks over time, which was assessed as the concentration of networks formed by UEIs, and determined by dividing the observed number of UEI1-UEI2 bindings by the theoretically possible number of UEI1-UEI2 bindings (the observed number of bindings/the theoretically possible number of bindings). It was demonstrated that the concentration of the networks increase over time (Fig. 11B). These results indicate that multifactorial interactions increase by activation of B cells. These results demonstrate that the method of the present invention is able to capture increase in multifactorial interactions due to increase in protein synthesis resulting from B cell activation.

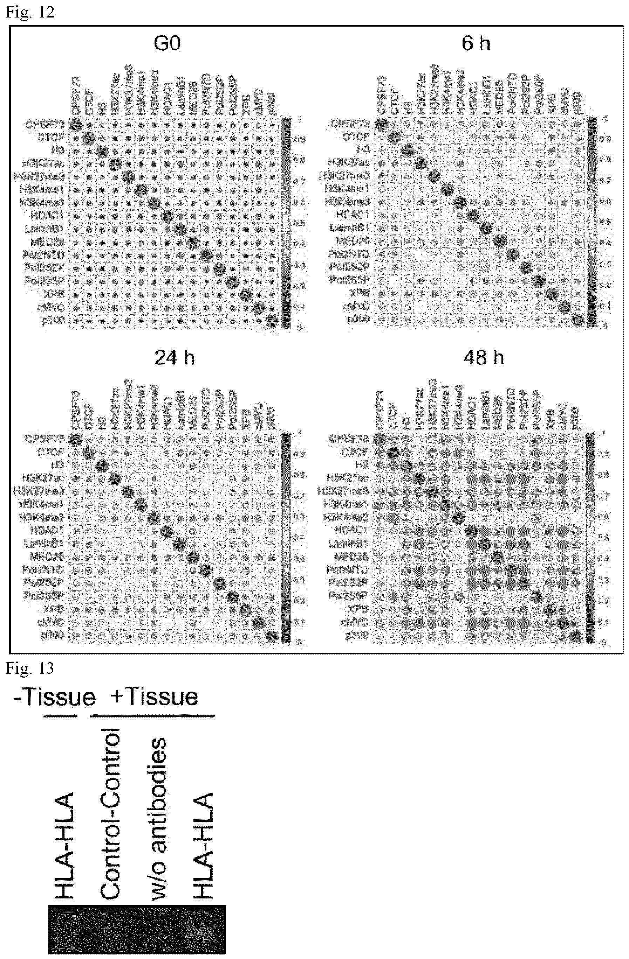

The number of bindings of antibody molecules per UEI was also analyzed for 16 types of analytical target factors, and the results are shown in Fig. 12. Correlation intensities are indicated by color scales, in which blue represents a correlation coefficient of zero, red represents a correlation coefficient of 1, and white represents a correlation coefficient of a mid-value (0.5). The dot size indicates the number of antibody bindings per UEI. These results indicate that the number of antibody bindings per UEI increases over time after LPS stimulation. The coefficients of positive correlations between the analytical target factors as indicated by the color scale indicate that almost all dots were blue immediately after LPS stimulation, but most of the dots turned into red 48 hours later. These results demonstrate that the correlations with other factors are enhanced in many of the analytical target factors over time after LPS stimulation. These results indicate that the method of the present invention is able to capture changes in the interactions between proteins as changes in the numbers and types of antibodies that bind to UEI. Further, it was demonstrated that the method of the present invention is able to simultaneously analyze multifactorial interactions of at least 16 different analytical target factors, and to assess changes in multifactorial interactions over time for at least 48 hours.

### Example 4: Identification of multifactorial interactions by using human renal tissues

### 1. Materials and methods

### 1-1 Production of DNA barcode-conjugated antibodies

### Preparation of biotinylated DNA barcodes

DNA barcodes having antibody barcode sequences, UMI sequences and adapter sequences for sequencing were designed for factors that are known to interact with a membrane protein HLA in human renal tissues, and the DNA barcodes were synthesized by a known technique in the same manner as in Examples 1 to 3. The 5' ends of the DNA barcodes were biotinylated by a conventional method, and the DNA barcodes were diluted in Tris-EDTA buffer to 100 µM, heated at 55°C for 10 minutes, and centrifuged at 18000xg at room temperature for 5 minutes. The concentration in the supernatant was checked and adjusted again, if necessary, to 100 µM. The analytical target factors were as follows: CD152 (CTLA-4), CD86, CD80, CD28, CD279 (PD-1), CD274 (B7-H1/PD-L1), CD134 (OX40), CD252 (OX40L), HLA-A/B/C, CD3, CD8, HLA-DR/DP/DQ, CD223 (LAG-3), CD4, CD314 (NKG2D), MICA/MICB, CD11b (activated), CD11c, TCR α/β, and TCR γ/δ. The sequences of the DNA barcodes are shown below. The antibody barcode sequences are underlined.
- target 1 oligo (5biotin)_BC1 (DNA barcode for anti-CD152 (CTLA-4) antibody): 
GACGCTCTTCCGATCTNNNNNNNWWSNNNWWWNNNNSACCGTGATGCGTA
AGTTTAGGACACCTCA
(SEQ ID NO: 49)
- target 1 oligo (5biotin)_BC2 (DNA barcode for anti-CD86 antibody): 
GACGCTCTTCCGATCTNNNNNNNWWSNNNWWWNNNNSACACATCGGCGTA
AGTTTAGGACACCTCA
(SEQ ID NO: 50)
- target 1 oligo (5biotin)_BC3 (DNA barcode for anti-CD80 antibody): 
GACGCTCTTCCGATCTNNNNNNNWWSNNNWWWNNNNSACGCCTAAGCGTA
AGTTTAGGACACCTCA
(SEQ ID NO: 51)
- target 1 oligo (5biotin)_BC4 (DNA barcode for anti-CD28 antibody): 
GACGCTCTTCCGATCTNNNNNNNWWSNNNWWWNNNNSACTGGTCAGCGTA
AGTTTAGGACACCTCA
(SEQ ID NO: 52)
- target 1 oligo (5biotin)_BC5 (DNA barcode for anti-CD279 (PD-1) antibody): 
GACGCTCTTCCGATCTNNNNNNNWWSNNNWWWNNNNSACCACTGTGCGTA
AGTTTAGGACACCTCA
(SEQ ID NO: 53)
- target 1 oligo (5biotin)_BC6 (DNA barcode for anti-CD274 (B7-H1/PD-L1) antibody): 
GACGCTCTTCCGATCTNNNNNNNWWSNNNWWWNNNNSACATTGGCGCGTA
AGTTTAGGACACCTCA
(SEQ ID NO: 54)
- target 1 oligo (5biotin)_BC7 (DNA barcode for anti-CD134 (OX40) antibody): 
GACGCTCTTCCGATCTNNNNNNNWWSNNNWWWNNNNSACGATCTGGCGTA
AGTTTAGGACACCTCA
(SEQ ID NO: 55)
- target 1 oligo (5biotin)_BC8 (DNA barcode for anti-CD252 (OX40L) antibody): 
GACGCTCTTCCGATCTNNNNNNNWWSNNNWWWNNNNSACTCAAGTGCGTA
AGTTTAGGACACCTCA
(SEQ ID NO: 56)
- target 1 oligo (5biotin)_BC9 (DNA barcode for anti-HLA-A/B/C antibody): 
GACGCTCTTCCGATCTNNNNNNNWWSNNNWWWNNNNSACCTGATCGCGTA
AGTTTAGGACACCTCA
(SEQ ID NO: 57)
- target 1 oligo (5biotin)_BC10 (DNA barcode for anti-CD3 antibody): 
GACGCTCTTCCGATCTNNNNNNNWWSNNNWWWNNNNSACAAGCTAGCGTA
AGTTTAGGACACCTCA
(SEQ ID NO: 58)
- target 1 oligo (5biotin)_BC11 (DNA barcode for anti-CD8 antibody): 
GACGCTCTTCCGATCTNNNNNNNWWSNNNWWWNNNNSACGTAGCCGCGTA
AGTTTAGGACACCTCA
(SEQ ID NO: 59)
- target 1 oligo (5biotin)_BC12 (DNA barcode for anti-HLA-DR/DP/DQ antibody): 
GACGCTCTTCCGATCTNNNNNNNWWSNNNWWWNNNNSACTACAAGGCGTA
AGTTTAGGACACCTCA
(SEQ ID NO: 60)
- target 1 oligo (5biotin)_BC13 (DNA barcode for anti-CD223 (LAG-3) antibody): 
GACGCTCTTCCGATCTNNNNNNNWWSNNNWWWNNNNSACTGTTGACTGCG
TAAGTTTAGGACACCTCA
(SEQ ID NO: 61)
- target 1 oligo (5biotin)_BC14 (DNA barcode for anti-CD4 antibody): 
GACGCTCTTCCGATCTNNNNNNNWWSNNNWWWNNNNSACACGGAACTGC
GTAAGTTTAGGACACCTCA
(SEQ ID NO: 62)
- target 1 oligo (5biotin)_BC15 (DNA barcode for anti-CD314 (NKG2D) antibody): 
GACGCTCTTCCGATCTNNNNNNNWWSNNNWWWNNNNSACTCTGACATGCG
TAAGTTTAGGACACCTCA
(SEQ ID NO: 63)
- target 1 oligo (5biotin)_BC16 (DNA barcode for anti-MICA/MICB antibody): 
GACGCTCTTCCGATCTNNNNNNNWWSNNNWWWNNNNSACGGACGGGCGT
AAGTTTAGGACACCTCA
(SEQ ID NO: 64)
- target 1 oligo (5biotin)_BC18 (DNA barcode for anti-CD11b (activated) antibody): 
GACGCTCTTCCGATCTNNNNNNNWWSNNNWWWNNNNSACGTGCGGACGC
GTAAGTTTAGGACACCTCA
(SEQ ID NO: 65)
- target 1 oligo (5biotin)_BC19 (DNA barcode for anti-CD11c antibody): 
GACGCTCTTCCGATCTNNNNNNNWWSNNNWWWNNNNSACCGTTTCACGCG
TAAGTTTAGGACACCTCA
(SEQ ID NO: 66)
- target 1 oligo (5biotin)_BC20 (DNA barcode for anti-TCRα/β antibody): 
GACGCTCTTCCGATCTNNNNNNNWWSNNNWWWNNNNSACAAGGCCACGC
GTAAGTTTAGGACACCTCA
(SEQ ID NO: 67)
- target 1 oligo (5biotin)_BC21 (DNA barcode for anti-TCRγ/δ antibody): 
GACGCTCTTCCGATCTNNNNNNNWWSNNNWWWNNNNSACTCCGAAACGC
GTAAGTTTAGGACACCTCA
(SEQ ID NO: 68)
- target 2 oligo (5biotin)_BC17 (DNA barcode for anti-CD152 (CTLA-4) antibody): 
TGTGCTCTTCCGATCTNNNNNNNWWSNNNWWWNNNNSACCTCTACGCGAT
ATGTTGAGACCACTCC
(SEQ ID NO: 69)
- target 2 oligo (5biotin)_BC24 (DNA barcode for anti-CD86 antibody): 
TGTGCTCTTCCGATCTNNNNNNNWWSNNNWWWNNNNSACGCTACCGCGAT
ATGTTGAGACCACTCC
(SEQ ID NO: 70)
- target 2 oligo (5biotin)_BC26 (DNA barcode for anti-CD80 antibody): 
TGTGCTCTTCCGATCTNNNNNNNWWSNNNWWWNNNNSACGCTCATGCGAT
ATGTTGAGACCACTCC
(SEQ ID NO: 71)
- target 2 oligo (5biotin)_BC28 (DNA barcode for anti-CD28 antibody): 
TGTGCTCTTCCGATCTNNNNNNNNWWSNNNWWWNNNNSACCTTTGGCGAT
ATGTTGAGACCACTCC
(SEQ ID NO: 72)
- target 2 oligo (5biotin)_BC29 (DNA barcode for anti-CD279 (PD-1) antibody): 
TGTGCTCTTCCGATCTNNNNNNNWWSNNNWWWNNNNSACTAGTTGGCGAT
ATGTTGAGACCACTCC
(SEQ ID NO: 73)
- target 2 oligo (5biotin)_BC31 (DNA barcode for anti-CD274 (B7-H1/PD-L1) antibody): 
TGTGCTCTTCCGATCTNNNNNNNWWSNNNWWWNNNNSACATCGTGGCGAT
ATGTTGAGACCACTCC
(SEQ ID NO: 74)
- target 2 oligo (5biotin)_BC32 (DNA barcode for anti-CD134 (OX40) antibody): 
TGTGCTCTTCCGATCTNNNNNNNWWSNNNWWWNNNNSACTGAGTGGCGAT
ATGTTGAGACCACTCC
(SEQ ID NO: 75)
- target 2 oligo (5biotin)_BC33 (DNA barcode for anti-CD252 (OX40L) antibody): 
TGTGCTCTTCCGATCTNNNNNNNWWSNNNWWWNNNNSACCGCCTGGCGAT
ATGTTGAGACCACTCC
(SEQ ID NO: 76)
- target 2 oligo (5biotin)_BC35 (DNA barcode for anti-HLA-A/B/C antibody): 
TGTGCTCTTCCGATCTNNNNNNNWWSNNNWWWNNNNSACAAAATGGCGAT
ATGTTGAGACCACTCC
(SEQ ID NO: 77)
- target 2 oligo (5biotin)_BC36 (DNA barcode for anti-CD3 antibody): 
TGTGCTCTTCCGATCTNNNNNNNWWSNNNWWWNNNNSACTGTTGGGCGAT
ATGTTGAGACCACTCC
(SEQ ID NO: 78)
- target 2 oligo (5biotin)_BC42 (DNA barcode for anti-CD8 antibody): 
TGTGCTCTTCCGATCTNNNNNNNWWSNNNWWWNNNNSACCGATTAGCGAT
ATGTTGAGACCACTCC
(SEQ ID NO: 79)
- target 2 oligo (5biotin)_BC30 (DNA barcode for anti-HLA-DR/DP/DQ antibody): 
TGTGCTCTTCCGATCTNNNNNNNWWSNNNWWWNNNNSACCCGGTGGCGAT
ATGTTGAGACCACTCC
(SEQ ID NO: 80)
- target 2 oligo (5biotin)_BC37 (DNA barcode for anti-CD223 (LAG-3) antibody): 
TGTGCTCTTCCGATCTNNNNNNNWWSNNNWWWNNNNSACATTCCGGCGAT
ATGTTGAGACCACTCC
(SEQ ID NO: 81)
- target 2 oligo (5biotin)_BC38 (DNA barcode for anti-CD4 antibody): 
TGTGCTCTTCCGATCTNNNNNNNWWSNNNWWWNNNNSACAGCTAGGCGAT
ATGTTGAGACCACTCC
(SEQ ID NO: 82)
- target 2 oligo (5biotin)_BC39 (DNA barcode for anti-CD314 (NKG2D) antibody): 
TGTGCTCTTCCGATCTNNNNNNNWWSNNNWWWNNNNSACGTATAGGCGAT
ATGTTGAGACCACTCC
(SEQ ID NO: 83)
- target 2 oligo (5biotin)_BC40 (DNA barcode for anti-MICA/MICB antibody): 
TGTGCTCTTCCGATCTNNNNNNNWWSNNNWWWNNNNSACTGGATCACGCG
ATATGTTGAGACCACTCC
(SEQ ID NO: 84)
- target 2 oligo (5biotin)_BC41 (DNA barcode for anti-CD11b (activated) antibody): 
TGTGCTCTTCCGATCTNNNNNNNWWSNNNWWWNNNNSACGTCGTCGCGAT
ATGTTGAGACCACTCC
(SEQ ID NO: 85)
- target 2 oligo (5biotin)_BC43 (DNA barcode for anti-CD11c antibody): 
TGTGCTCTTCCGATCTNNNNNNNWWSNNNWWWNNNNSACGCTGTAGCGAT
ATGTTGAGACCACTCC
(SEQ ID NO: 86)
- target 2 oligo (5biotin)_BC44 (DNA barcode for anti-TCRα/β antibody): 
TGTGCTCTTCCGATCTNNNNNNNWWSNNNWWWNNNNSACATTATAGCGATA
TGTTGAGACCACTCC
(SEQ ID NO: 87)
- target 2 oligo (5biotin)_BC45 (DNA barcode for anti-TCRγ/δ antibody):

TGTGCTCTTCCGATCTNNNNNNNWWSNNNWWWNNNNSACGAATGAGCGAT
ATGTTGAGACCACTCC
(SEQ ID NO: 88)

### Preparation of antibody-streptavidin complexes

The following mouse monoclonal antibodies (BioLegend) were labeled with streptavidin in the same manner as in Example 1: anti-CD152 (CTLA-4) antibody (Cat. No. 369602), anti-human CD80 antibody (Cat. No. 305202), anti-human CD86 antibody (Cat. No. 305402), anti-human CD28 antibody (Cat. No. 302902), anti-human CD279 (PD-1) antibody (Cat. No. 329902), anti-human CD274 (B7-H1, PD-L1) antibody (Cat. No. 329702), anti-human CD134 (OX40) antibody (Cat. No. 326302), anti-human HLA-A/B/C antibody (Cat. No. 311402), anti-human CD3 antibody (Cat. No. 300402), anti-human CD8 antibody (Cat. No. 344702), anti-human HLA-DR/DP/DQ antibody (Cat. No. 361702), anti-human CD223 (LAG-3) antibody (Cat. No. 369302), anti-human CD4 antibody (Cat. No. 344602), anti-human CD314 (NKG2D) antibody (Cat. No. 320802), anti-human MICA/MICB antibody (Cat. No. 320902), anti-human CD11b (activated) antibody (Cat. No. 301402), anti-human CD11c antibody (Cat. No. 301602), anti-human TCR α/β antibody (Cat. No. 306702), and anti-human TCR γ/δ antibody (Cat. No. 331202).

### Preparation of DNA barcode-labeled antibodies

The streptavidin labeled-antibodies were allowed to bind to the corresponding biotinylated DNA barcodes to form conjugates in the same manner as in Example 1. The combinations of the antibodies and the DNA barcodes are as follows: anti-CD152 (CTLA-4) antibody-target 1 oligo (5biotin)_BC1 (SEQ ID NO: 49), anti-CD86 antibody-target 1 oligo (5biotin)_BC2 (SEQ ID NO: 50), anti-CD80 antibody-target 1 oligo (5biotin)_BC3 (SEQ ID NO: 51), anti-CD28 antibody-target 1 oligo (5biotin)_BC4 (SEQ ID NO: 52), anti-CD279 (PD-1) antibody-target 1 oligo (5biotin)_BC5 (SEQ ID NO: 53), anti-CD274 (B7-H1/PD-L1) antibody-target 1 oligo (5biotin)_BC6 (SEQ ID NO: 54), anti-CD134 (OX40) antibody-target 1 oligo (5biotin)_BC7 (SEQ ID NO: 55), anti-CD252 (OX40L) antibody-target 1 oligo (5biotin)_BC8 (SEQ ID NO: 56), anti-HLA-A/B/C antibody-target 1 oligo (5biotin)_BC9 (SEQ ID NO: 57), anti-CD3 antibody-target 1 oligo (5biotin)_BC10 (SEQ ID NO: 58), anti-CD8 antibody-target 1 oligo (5biotin)_BC11 (SEQ ID NO: 59), anti-HLA-DR/DP/DQ antibody-target 1 oligo (5biotin)_BC12 (SEQ ID NO: 60), anti-CD223 (LAG-3) antibody-target 1 oligo (5biotin)_BC13 (SEQ ID NO: 61), anti-CD4 antibody-target 1 oligo (5biotin)_BC14 (SEQ ID NO: 62), anti-CD314 (NKG2D) antibody-target 1 oligo (5biotin)_BC15 (SEQ ID NO: 63), anti-MICA/MICB antibody-target 1 oligo (5biotin)_BC16 (SEQ ID NO: 64), anti-CD11b (activated) antibody-target 1 oligo (5biotin)_BC18 (SEQ ID NO: 65), anti-CD11c antibody-target 1 oligo (5biotin)_BC19 (SEQ ID NO: 66), anti-TCRα/β antibody-target 1 oligo (5biotin)_BC20 (SEQ ID NO: 67), anti-TCRγ/δ antibody-target 1 oligo (5biotin)_BC21 (SEQ ID NO: 68), anti-CD152 (CTLA-4) antibody-target 2 oligo (5biotin)_BC17 (SEQ ID NO: 69), anti-CD86 antibody-target 2 oligo (5biotin)_BC24 (SEQ ID NO: 70), anti-CD80 antibody-target 2 oligo (5biotin)_BC26 (SEQ ID NO: 71), anti-CD28 antibody-target 2 oligo (5biotin) _BC28 (SEQ ID NO: 72), anti-CD279 (PD-1) antibody-target 2 oligo (5biotin)_BC29 (SEQ ID NO: 73), anti-CD274 (B7-H1/PD-L1) antibody-target 2 oligo (5biotin)_BC31 (SEQ ID NO: 74), anti-CD134 (OX40) antibody-target 2 oligo (5biotin)_BC32 (SEQ ID NO: 75), anti-CD252 (OX40L) antibody-target 2 oligo (5biotin)_BC33 (SEQ ID NO: 76), anti-HLA-A/B/C antibody-target 2 oligo (5biotin)_BC35 (SEQ ID NO: 77), anti-CD3 antibody-target 2 oligo (5biotin)_BC36 (SEQ ID NO: 78), anti-CD8 antibody-target 2 oligo (5biotin)_BC42 (SEQ ID NO: 79), anti-HLA-DR/DP/DQ antibody-target 2 oligo (5biotin)_BC30 (SEQ ID NO: 80), anti-CD223 (LAG-3) antibody-target 2 oligo (5biotin)_BC37 (SEQ ID NO: 81), anti-CD4 antibody-target 2 oligo (5biotin)_BC38 (SEQ ID NO: 82), anti-CD314 (NKG2D) antibody-target 2 oligo (5biotin)_BC39 (SEQ ID NO: 83), anti-MICA/MICB antibody-target 2 oligo (5biotin)_BC40 (SEQ ID NO: 84), anti-CD11b (activated) antibody-target 2 oligo (5biotin)_BC41 (SEQ ID NO: 85), anti-CD11c antibody-target 2 oligo (5biotin)_BC43 (SEQ ID NO: 86), anti-TCRα/β antibody-target 2 oligo (5biotin)_BC44 (SEQ ID NO: 87), and anti-TCRγ/δ antibody-target 2 oligo (5biotin)_BC45 (SEQ ID NO: 88). The solvent of the solutions containing one of the DNA barcode-labeled antibodies after the reaction was exchanged with PBS by a conventional method, and the total volume was adjusted to 30 µL with PBS.

The DNA barcode-labeled antibodies prepared as above were run by electrophoresis on a 4% TAE gel for 10 minutes. The gel was then treated with ethidium bromide for 10 minutes to visualize the bands to examine the conjugation of the DNA barcodes. As controls, 50 pmol of the DNA barcodes were run, and the relative band intensities were determined for all bands appeared on the lanes of the DNA barcode-labeled antibody samples by image analysis. The amounts of the DNA barcodes in the prepared DNA barcode-labeled antibodies were estimated. Samples for which successful conjugation of the DNA barcodes was confirmed were stored at 4°C after addition of 50 mg/mL BSA and 1% sodium azide at final concentrations of 1 µg/µL and 0.05%, respectively.

### 1-2 Preparation of specimens

Renal cancer tissue and the peripheral normal renal tissue were harvested from a human patient with renal cancer. The specimens were fixed in 4% paraformaldehyde/PBS with shaking at 4°C overnight. After fixation, the specimens were dehydrated in 15% sucrose with shaking at 4°C for one day, and then in 30% sucrose with shaking at 4°C for one day. After dehydration, the specimens were embedded in O.C.T. compound (Sakura Finetek Japan), and frozen and sectioned into thin slices. The compound was removed from the sections using PBS, and placed in each well of fluororesin-coated glass slides (super hydrophobic printed glass slides, 10 wells, 6 mm diameter, Matsunami Glass Ind., Ltd., Cat. No. TF1006, see Fig. 6). Then, 25 µL of TrueBlack IF Background Suppressor sol.B (trade name) (BIOTIUM, Cat. No. 23012) was added to each well and incubated at room temperature for 15 minutes to block and permeate the sections.

Then, 25 µL of a mixture of the DNA barcode-labeled antibodies prepared in the above 1-1 or a mixture of the DNA barcodes was added to each section, and allowed to contact with the section at 4°C for 30 minutes. The section was washed 3 times with PBS containing 0.1% Tween20 and then washed 5 times with PBS, and 40 µL of 4% paraformaldehyde/PBS was added to each well and allowed to stand at room temperature for 10 minutes for post-fixation. The post-fixed section was washed 3 times with PBS, and stored at 4°C with addition of 50 µL of PBS.

### 1-3 Joining PCR and next-generation sequencer analysis

The PCR reaction solution, at a volume of 20 µL, prepared in the same manner as in Example 1 was added to the specimens prepared in the above 1-2 and allowed to solidify into a gel, and frame-seal slide chambers and plastic covers were placed on the glass slides. Joining PCR of the antibody barcodes in close proximity was performed in the same manner as in Example 2. The PCR products were extracted and purified to prepare libraries for sequencing. Tagging PCR was performed and the PCR products were extracted. Sequencing was performed using HiSeq system of Illumina in the same manner as in Example 2.

### 2. Results

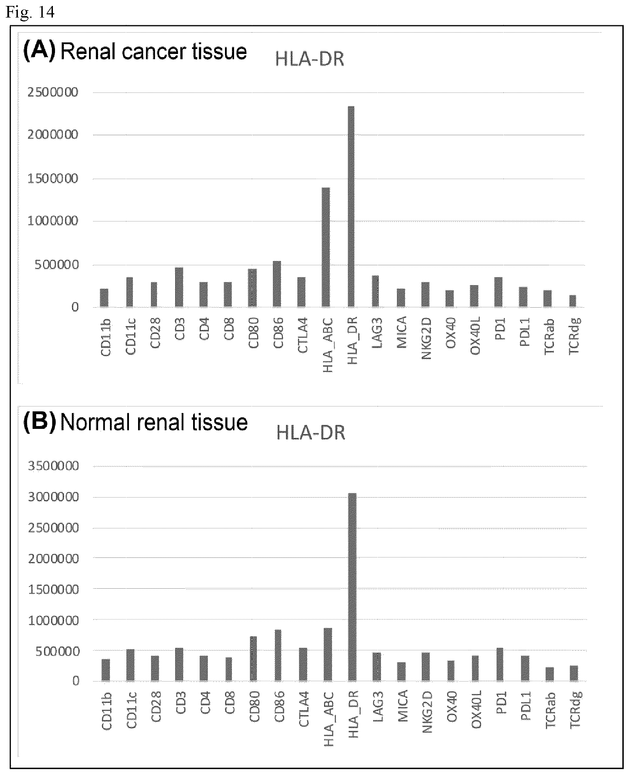

The results of gel electrophoresis after the PCR reaction are shown in Fig. 13. Bands of PCR products were not detected in the absence of tissues or when IgG as control was used or in the absence of antibodies, confirming that the method of the present invention is able to specifically detect multifactorial interactions even in human tissues used as a biological sample (Fig. 13). Fig. 14 shows interactions between the membrane protein (HLA-DR) and 20 types of analytical target factors detected in the human renal cancer tissue (Fig. 14A) and the human normal renal tissue (Fig. 14B). The vertical axis indicates the number of sequencing reads. The interactions between the membrane protein (HLA-DR) and 20 types of analytical target factors detected in the human renal cancer tissue and the human normal renal tissue revealed marked increase in interactions between HLA-DR and HLA-A/B/C in the renal cancer tissue. HLA complexes expressed on cell surfaces are known to contribute to the immune surveillance by T cells, and the T cell receptor binds to the complexes of HLA class I and HLA class II (Armony G et al., Molecular Immunology 136 (2021), pp.16-25). Thus, the results suggest that binding of HLA-DR (HLA class II) and HLA-A/B/C (HLA class I) may control the recognition of cancer cells by T cells. It was demonstrated that the method of the present invention is useful as an unprecedented assessment tool for diagnosis of cancer. These results further demonstrate that the method of the present invention is able to simultaneously capture multifactorial interactions of at least 20 types of analytical target factors.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The scientific literature and the patent literature cited herein are hereby incorporated by reference in their entirety.

## Claims

1. A method for identifying multifactorial interactions in a biological sample, comprising the following steps (a) to (f):
(a) selecting a plurality of analytical target factors,
(b) labeling antibody molecules against each of the selected factors with DNA barcodes,
(c) contacting a biological sample containing the analytical target factors with the antibody molecules labeled with the DNA barcodes,
(d) embedding the biological sample in a hydrogel after step (c),
(e) performing, within the hydrogel, a nucleic acid amplification reaction using a plurality of primers designed such that, when two antibodies come in close proximity, the primers produce an amplification product containing both DNA barcode sequences from the antibodies, and
(f) analyzing a nucleotide sequence of the produced amplification product to detect a presence of a factor that simultaneously interacts with two or more factors.

2. The method according to claim 1, wherein the DNA barcodes each comprise a sequence for distinguishing different antibody types or distinguishing different antibody types and different DNA barcode types, and a sequence for distinguishing individual antibody molecules.

3. The method according to claim 1, wherein the plurality of primers each comprise a primer set for joining the DNA barcode sequences of two antibodies that come in close proximity, and a primer set for amplifying the joined DNA barcode sequences.

4. The method according to claim 3, wherein the primers of the primer sets for the joining each comprise a 5' end sequence that hybridizes to its paired primer, a 3' end sequence that hybridizes to a 3' region of one of the DNA barcode sequences, and a sequence for distinguishing individual primer molecules.

5. The method according to claim 3, wherein the primers of the primer set for the amplification each comprise a sequence that hybridizes to a 5' region of one of the DNA barcode sequences.

6. The method according to claim 3, wherein the primer set for the joining is immobilized on a polymer that forms the hydrogel.

7. The method according to any one of claims 1 to 6, wherein the nucleic acid amplification reaction is performed on a glass slide.

8. The method according to any one of claims 1 to 6, wherein the nucleic acid amplification reaction is PCR.

9. The method according to any one of claims 1 to 6, wherein the analysis of the nucleotide sequence of the amplification product is performed using a next-generation sequencer.

10. The method according to claim 9, wherein the primers are designed to produce an amplification product that is shorter than a maximum read length of the next-generation sequencer to be used.

11. The method according to any one of claims 1 to 6, wherein the biological sample is a human biological sample.

12. A kit for identifying multifactorial interactions in a biological sample, comprising DNA barcode-labeled antibodies against analytical target factors; primers designed to produce an amplification product formed from joined DNA barcode sequences from two antibodies by a nucleic acid amplification reaction; and a hydrogel.
